# EUROPÄISCHE PATENTANMELDUNG

(11) **EP 2 298 272 A2**
(43) Veröffentlichungstag der Anmeldung: **23.03.2011**
(21) Anmeldenummer: 10170083.9
(22) Anmeldetag: 20.07.2010
(51) Int. Cl.: A61K 8/04, A61Q 15/00, B65D 83/14

(54) **Antitranspirant-Sprays**

(30) Priorität: 19.08.2009 DE 102009028651
(71) Anmelder: Henkel AG & Co. KGaA, 40589 Düsseldorf (DE)
(72) Erfinder: Banowski, Bernhard, 40597, Düsseldorf (DE); Claas, Marcus, 40723, Hilden (DE); Park, Theodor, 22549, Hamburg (DE)

(57) **Zusammenfassung**

Antitranspirant-Suspensionen können auch mit einem höheren Gehalt an protischen Lösungsmitteln aus Druckbehältern mit Hilfe eines Treibmittels versprüht werden, wenn eine Kombination aus einer treibmittelhaltigen Antitranspirant-Zusammensetzung und einem dafür geeigneten Spender eingesetzt wird, bei der die treibmittelhaltige Antitranspirant-Zusammensetzung bezogen auf ihr Gewicht mindestens 1 Gew.-% mindestens eines Antitranspirant-Wirkstoffes enthält, mindestens 10 Gew.-% eines oder mehrerer protischer Lösungsmittel enthält, 5 bis 60 Gew.-% Treibmittel enthält, und der Spender einen Druckbehälter aus Metall umfaßt, welcher eine Pulverlack-Innenbeschichtung aufweist.

## Beschreibung

Gegenstand der vorliegenden Erfindung sind schweißhemmende Zusammensetzungen, insbesondere auf Basis einer treibmittelfreien oder mit einem Treibmittel versprühbaren Suspension, die eine vergrößerte Formulierbarkeit und Formulierungsfreiheit des schweißhemmenden Wirkstoffs ermöglichen.

Üblicherweise enthalten mit einem Treibmittel versprühbare Antitranspirant-Suspensionen neben schweißreduzierenden Wirkstoffen in der Regel mindestens ein kosmetisches Öl als Träger für den teilchenförmigen schweißreduzierenden Wirkstoff und werden wasserfrei formuliert. Dieser Verzicht auf Wasser in den zu versprühenden Formulierungen ist bei herkömmlichen Aluminium-oder Weißblech-Druckbehältern angeraten, da die Behälter durch wasserhaltige Suspensionen schweißhemmender Salze angegriffen werden und ein schlagartiger Druckverlust durch Behälterkorrosion nicht ausgeschlossen werden kann. Es hat Versuche gegeben, Schutzlacke auf der Basis von PVC oder PAM auf die Innenseiten der Dosen aufzutragen. Einerseits werden bei der Auftragung dieser Lacke ökologisch oder toxikologisch bedenkliche Lösungsmittel eingesetzt, andererseits werden die vorstehend genannten Probleme nicht vollständig gelöst, d.h. auch die Lackbeschichtungen sind nicht vollständig stabil gegen wasserhaltige Suspensionen von Antitranspirantsalzen.

Die Formulierungsfreiheit für das zu versprühende Produkt wird durch den Verzicht auf Wasser oder andere protische Lösungsmittel stark eingeengt. Es ist wünschenswert, auch wasserhaltige Formulierungen oder Formulierungen mit protischen Lösungsmittel in der konvenienten Applikationsform Aerosol anbieten zu können.

Es wurde nun überraschend gefunden, daß speziell konfektionierte Mittel in einem darauf abgestimmten Druckbehälter verpackt werden können und die oben genannten Probleme nicht zeigen.

Gegenstand der vorliegenden Erfindung ist in einer ersten Ausführungsform eine Kombination aus einer treibmittelhaltigen Antitranspirant-Zusammensetzung und einem dafür geeigneten Spender, bei der
A) die treibmittelhaltige Antitranspirant-Zusammensetzung bezogen auf ihr Gewicht
   a1) mindestens 1 Gew.-% mindestens eines Antitranspirant-Wirkstoffes enthält,
   a2) mindestens 10 Gew.-% eines oder mehrerer protischer Lösungsmittel enthält,
   a3) 5 bis 60 Gew.-% Treibmittel enthält, und
B) der Spender einen Druckbehälter aus Metall umfaßt, welcher eine Pulverlack-Innenbeschichtung aufweist.

Die erfindungsgemäße schweißhemmende Wirkstoffkombination eignet sich bevorzugt für versprühbar konfektionierte Zusammensetzungen, insbesondere für treibmittelhaltige Suspensionen, die als Antitranspirant-Spray angewendet werden. Die erfindungsgemäße Kombination mit dem speziellen Druckbehälter ermöglicht Produkte mit breiter Formulierungsfreiheit und den Vorteilen der Aerosolapplikation. "Normalbedingungen" sind im Sinne der vorliegenden Anmeldung eine Temperatur von 20 °C und ein Druck von 1013,25 mbar. Schmelzpunktangaben beziehen sich ebenfalls auf einen Druck von 1013,25 mbar.

Die erfindungsgemäßen Zusammensetzungen enthalten als Inhaltsstoff a1) mindestens einen Antitranspirant-Wirkstoff.

Bevorzugte Antitranspirant-Wirkstoffe sind ausgewählt aus den wasserlöslichen adstringierenden anorganischen und organischen Salzen des Aluminiums und Zinks bzw. beliebigen Mischungen dieser Salze. Alumosilicate und Zeolithe zählen erfindungsgemäß nicht zu den Antitranspirant-Wirkstoffen.

Erfindungsgemäß wird unter Wasserlöslichkeit eine Löslichkeit von wenigstens 5 Gew.-% bei 20 °C verstanden, das heißt, dass Mengen von wenigstens 5 g des Antitranspirant-Wirkstoffs in 95 g Wasser bei 20 °C löslich sind.

Besonders bevorzugte Antitranspirant-Wirkstoffe sind ausgewählt aus Aluminiumchlorhydrat, insbesondere Aluminiumchlorhydrat mit der allgemeinen Formel [Al₂(OH)₅Cl · 1-6 H₂O]ₙ, bevorzugt [Al₂(OH)₅Cl · 2-3 H₂O]ₙ, das in nicht-aktivierter oder in aktivierter (depolymerisierter) Form vorliegen kann, sowie Aluminiumchlorhydrat mit der allgemeinen Formel [Al₂(OH)₄Cl₂ · 1-6 H₂O]ₙ, bevorzugt [Al₂(OH)₄Cl₂ · 2-3 H₂O]ₙ, das in nicht-aktivierter oder in aktivierter (depolymerisierter) Form vorliegen kann.

Weiterhin bevorzugt sind Aluminiumsesquichlorhydrat, Aluminiumdichlorhydrat, Aluminiumchlorohydrex-Propylenglycol (PG) oder Aluminiumchlorohydrex-Polyethylenglycol (PEG), Aluminium-Glycol-Komplexe, z. B. Aluminium-Propylenglycol-Komplexe, Aluminiumsesquichlorohydrex-PG oder Aluminiumsesquichlorohydrex-PEG, Aluminium-PG-dichlorohydrex oder Aluminium-PEGdichlorohydrex, Aluminiumhydroxid, weiterhin ausgewählt aus Kaliumaluminiumsulfat (KAl(SO₄)₂ · 12 H₂O, Alaun), Aluminiumundecylenoylcollagenaminosäure, Natriumaluminiumlactat + Aluminiumsulfat, Natriumaluminiumchlorhydroxylactat, Aluminiumbromhydrat, Aluminiumchlorid, den Komplexen von Zink- und Natriumsalzen, den Aluminiumsalzen von Lipoaminosäuren, Aluminiumsulfat, Aluminiumlactat, Aluminiumchlorhydroxyallantoinat, Natrium-Aluminium-Chlorhydroxylactat, Zinkchlorid, Zinksulfocarbolat und Zinksulfat.

Erfindungsgemäß besonders bevorzugte Antitranspirant-Wirkstoffe sind ausgewählt aus so genannten "aktivierten" Aluminiumsalzen, die auch als Antitranspirant-Wirkstoffe "mit erhöhter Wirksamkeit (englisch: enhanced activity)" bezeichnet werden. Derartige Wirkstoffe sind im Stand der Technik bekannt und auch kommerziell erhältlich. Aktivierte Aluminiumsalze werden in der Regel durch Wärmebehandlung einer relativ verdünnten Lösung des Salzes erzeugt (z.B. etwa 10 Gew.-% Salz), um dessen HPLC-Peak 4-zu-Peak 3-Flächenverhältnis zu vergrößern. Das aktivierte Salz kann anschließend zu einem Pulver getrocknet, insbesondere sprühgetrocknet werden. Neben der Sprühtrocknung ist z. B. auch die Walzentrocknung geeignet.

Aktivierte Aluminiumsalze haben typischerweise ein HPLC-Peak 4-zu-Peak 3-Flächenverhältnis von mindestens 0,4, bevorzugt mindestens 0,7, besonders bevorzugt mindestens 0,9, wobei mindestens 70% des Aluminiums diesen Peaks zuzuordnen sind.

Aktivierte Aluminiumsalze müssen nicht notwendigerweise als sprühgetrocknetes Pulver eingesetzt werden.

Weitere bevorzugte schweißhemmende Wirkstoffe sind basische Calcium-Aluminiumsalze. Diese Salze werden durch Umsetzen von Calciumcarbonat mit Aluminiumchlorhydroxid oder Aluminiumchlorid und Aluminiumpulver oder durch Zusetzen von Calciumchlorid-Dihydrat zu Aluminiumchlorhydroxid hergestellt.

Weitere bevorzugte schweißhemmende Wirkstoffe sind aktivierte Aluminiumsalze enthalten 5 - 78 Gew.-% (USP) eines aktivierten schweißhemmenden Aluminiumsalzes, eine Aminosäure oder Hydroxyalkansäure in einer solchen Menge, um ein (Aminosäure oder Hydroxyalkansäure) zu Aluminium - Gewichtsverhältnis von 2:1 - 1:20 und bevorzugt 1:1 bis 1:10 bereitzustellen, sowie ein wasserlösliches Calciumsalz in einer solchen Menge, um ein Ca:Al-Gewichtsverhältnis von 1:1 - 1:28 und bevorzugt 1:2 - 1:25 bereitzustellen.

Besonders bevorzugte feste aktivierte schweißhemmende Salzzusammensetzungen enthalten 48 - 78 Gew.-% (USP), bevorzugt 66 - 75 Gew.-% eines aktivierten Aluminiumsalzes und 1 - 16 Gew.-%, bevorzugt 4 - 13 Gew.-% molekular gebundenes Wasser (Hydratationswasser), weiterhin soviel wasserlösliches Calciumsalz, dass das Ca:Al-Gewichtsverhältnis 1:1 - 1:28, bevorzugt 1:2 - 1:25, beträgt und soviel Aminosäure, dass das Aminosäure zu (AI+Zr) - Gewichtsverhältnis 2:1 - 1:20, bevorzugt 1:1 - 1:10, beträgt.

Weitere besonders bevorzugte feste schweißhemmende aktivierte Salzzusammensetzungen enthalten 48 - 78 Gew.-% (USP), bevorzugt 66 - 75 Gew.-% eines aktivierten Aluminiumsalzes und 1 - 16 Gew.-%, bevorzugt 4 - 13 Gew.-% molekular gebundenes Wasser (Hydratationswasser), weiterhin soviel wasserlösliches Calciumsalz, dass das Ca:Al-Gewichtsverhältnis 1:1 - 1:28, bevorzugt 1:2 - 1:25, beträgt und soviel Glycin, dass das Glycin zu Al - Gewichtsverhältnis 2:1 - 1:20, bevorzugt 1:1 - 1:10, beträgt.

Weitere besonders bevorzugte feste schweißhemmende aktivierte Salzzusammensetzungen enthalten 48 - 78 Gew.-% (USP), bevorzugt 66 - 75 Gew.-% eines aktivierten Aluminiumsalzes und 1 - 16 Gew.-%, bevorzugt 4 - 13 Gew.-% molekular gebundenes Wasser, weiterhin soviel wasserlösliches Calciumsalz, dass das Ca:Al-Gewichtsverhältnis 1:1 - 1:28, bevorzugt 1:2 - 1:25, beträgt und soviel Hydroxyalkansäure, dass das Hydroxyalkansäure zu Al - Gewichtsverhältnis 2:1 - 1:20, bevorzugt 1:1 - 1:10, beträgt.

Für die Stabilisierung der schweißhemmenden Salze bevorzugte wasserlösliche Calciumsalze sind ausgewählt aus Calciumchlorid, Calciumbromid, Calciumnitrat, Calciumcitrat, Calciumformiat, Calciumacetat, Calciumgluconat, Calciumascorbat, Calciumlactat, Calciumglycinat, Calciumcarbonat, Calciumsulfat, Calciumhydroxid, sowie Mischungen davon.

Für die Stabilisierung der schweißhemmenden Salze bevorzugte Aminosäuren sind ausgewählt aus Glycin, Alanin, Leucin, Isoleucin, β-Alanin, Valin, Cystein, Serin, Tryptophan, Phenylalanin, Methionin, β-Amino-n-butansäure und γ-Amino-n-butansäure und den Salzen davon, jeweils in der d-Form, der I-Form und der dl-Form; Glycin ist besonders bevorzugt.

Für die Stabilisierung der schweißhemmenden Salze bevorzugte Hydroxyalkansäuren sind ausgewählt aus Glycolsäure und Milchsäure.

Weitere bevorzugte schweißhemmende Wirkstoffe sind aktivierte Aluminiumsalze, enthaltend 5 - 78 Gew.-% (USP) eines aktivierten schweißhemmenden Aluminiumsalzes, eine Aminosäure oder Hydroxyalkansäure in einer solchen Menge, um ein (Aminosäure oder Hydroxyalkansäure) zu Al - Gewichtsverhältnis von 2:1 - 1:20 und bevorzugt 1:1 bis 1:10 bereitzustellen, sowie ein wasserlösliches Strontiumsalz in einer solchen Menge, um ein Sr:Al-Gewichtsverhältnis von 1:1 - 1:28 und bevorzugt 1:2 - 1:25 bereitzustellen.

Besonders bevorzugte feste schweißhemmende aktivierte Salzzusammensetzungen enthalten 48 - 78 Gew.-% (USP), bevorzugt 66 - 75 Gew.-% eines aktivierten Aluminiumsalzes und 1 - 16 Gew.-%, bevorzugt 4 - 13 Gew.-% molekular gebundenes Wasser, weiterhin soviel wasserlösliches Strontiumsalz, dass das Sr:Al-Gewichtsverhältnis 1:1 - 1:28, bevorzugt 1:2 - 1:25, beträgt und soviel Aminosäure, dass das Aminosäure zu Al - Gewichtsverhältnis 2:1 - 1:20, bevorzugt 1:1 - 1:10, beträgt.

Weitere besonders bevorzugte feste schweißhemmende aktivierte Salzzusammensetzungen enthalten 48 - 78 Gew.-% (USP), bevorzugt 66 - 75 Gew.-% eines aktivierten Aluminiumsalzes und 1 - 16 Gew.-%, bevorzugt 4 - 13 Gew.-% molekular gebundenes Wasser, weiterhin soviel wasserlösliches Strontiumsalz, dass das Sr:Al-Gewichtsverhältnis 1:1 - 1:28, bevorzugt 1:2 - 1:25, beträgt und soviel Glycin, dass das Glycin zu Al - Gewichtsverhältnis 2:1 - 1:20, bevorzugt 1:1 - 1:10, beträgt.

Weitere besonders bevorzugte feste schweißhemmende aktivierte Salzzusammensetzungen enthalten 48 - 78 Gew.-% (USP), bevorzugt 66 - 75 Gew.-% eines aktivierten Aluminiumsalzes und 1 - 16 Gew.-%, bevorzugt 4 - 13 Gew.-% molekular gebundenes Wasser, weiterhin soviel wasserlösliches Strontiumsalz, dass das Sr:Al-Gewichtsverhältnis 1:1 - 1:28, bevorzugt 1:2 - 1:25, beträgt und soviel Hydroxyalkansäure, dass das Hydroxyalkansäure zu Al - Gewichtsverhältnis 2:1 - 1:20, bevorzugt 1:1 - 1:10, beträgt.

Weitere bevorzugte aktivierte Aluminiumsalze sind solche der allgemeinen Formel Al₂(OH)₆₋ₐXa, worin X Cl, Br, I oder NO₃ ist und "a" ein Wert von 0,3 bis 5, bevorzugt von 0,8 bis 2,5 und besonders bevorzugt 1 bis 2 ist, so dass das Molverhältnis von Al:X 0,9:1 bis 2,1:1 beträgt. Bei diesen Salzen ist im Allgemeinen etwas Hydratationswasser assoziativ gebunden, typischerweise 1 bis 6 Mol Wasser pro Mol Salz. Besonders bevorzugt ist Aluminiumchlorhydrat (d.h. X ist Cl in der vorgenannten Formel) und speziell 5/6-basisches Aluminiumchlorhydrat, worin "a" 1 beträgt, so dass das Molverhältnis von Aluminium zu Chlor 1,9:1 bis 2,1:1 beträgt.

Weitere bevorzugte schweißhemmende Wirkstoffe sind in US 6663854 und US 20040009133 offenbart. Die schweißhemmenden Wirkstoffe liegen in ungelöster, suspendierter Form vor.

Sofern die schweißhemmenden Wirkstoffe in einem mit Wasser nicht mischbaren Träger suspendiert vorliegen, ist es aus Gründen der Produktstabilität bevorzugt, dass die Wirkstoffpartikel eine zahlenmittlere Partikelgröße von 0,1 - 200 µm, bevorzugt 1 - 50 µm, besonders bevorzugt 3 - 20 µm und außerordentlich bevorzugt 5 - 10 µm, aufweisen. Bevorzugte Wirkstoffpartikel weisen eine volumenmittlere Partikelgröße von 0,2 - 220 µm, bevorzugt 3 - 60 µm, besonders bevorzugt 4 - 25 µm, weiterhin bevorzugt 5 - 20 µm und außerordentlich bevorzugt 10 - 15,5 µm, auf. Bevorzugte Aluminiumsalze weisen ein molares Metall-zu-Chlorid-Verhältnis von 1,9 -2,1, bzw. für Sesquichlorohydrate von 1,5:1-1,8:1 auf.

Besonders bevorzugte erfindungsgemäße Zusammensetzungen sind dadurch gekennzeichnet, dass der mindestens eine Antitranspirant-Wirkstoff in einer Menge von 5 - 40 Gew.-%, bevorzugt 10 - 35 Gew.-%, besonders bevorzugt 11 - 28 Gew.-% und außerordentlich bevorzugt 12 - 20 Gew.-%, enthalten ist, bezogen auf das Gesamtgewicht der kristallwasserfreien Aktivsubstanz (USP) in der treibmittelfreien Gesamtzusammensetzung.

In einer besonders bevorzugten Ausführungsform enthält die Zusammensetzung ein adstringierendes Aluminiumsalz, insbesondere Aluminiumchlorohydrat, besonders bevorzugt Aluminiumchlorohydrat mit einer kristallwasserfreien Aktivsubstanz (USP) von 72 - 88 Gew.-%, bezogen auf den Rohstoff tel quel. Bevorzugte nicht-aktivierte Aluminiumchlorohydrate sind beispielsweise pulverförmig als Micro Dry^{®}, Micro Dry^{®} Ultrafine oder Micro Dry^{®}-323 von Summit/Reheis, als Chlorhydrol^{®} (Pulver) sowie in aktivierter Form als Reach^{®} 101, Reach^{®} 103, Reach^{®} 501 von Reheis/Summit oder AACH-7171 von Summit vertrieben wird. Unter dem Namen Reach^{®} 301 wird ein Aluminiumsesquichlorohydrat von Reheis angeboten, das auch besonders bevorzugt ist.

Die Konfektionierung der erfindungsgemäßen Zusammensetzungen, die als Spray appliziert werden, richtet sich vorzugsweise nach den Anforderungen der gewünschten Sprayapplikation.

Erfindungsgemäß bevorzugte Kombinationen sind **dadurch gekennzeichnet, daß** die treibmittelhaltige Antitranspirant-Zusammensetzung bezogen auf ihr Gewicht mindestens einen Antitranspirant-Wirkstoff in einer Gesamtmenge von 3 - 25 Gew.-%, vorzugsweise 5 - 22 Gew.-% und insbesondere 10 - 20 Gew.-%, bezogen auf das Gesamtgewicht der Aktivsubstanz in der gesamten Zusammensetzung, enthält.

Die erfindungsgemäßen Zusammensetzungen enthalten als Inhaltsstoff a2) bezogen auf ihr Gewicht mindestens 10 Gew.-% eines oder mehrerer protischer Lösungsmittel.

Im Gegensatz zu herkömmlichen Aerosol-Formulierungen, bei denen streng auf die Abwesenheit von Wasser und anderer protischer Lösungsmittel geachtet werden muß, enthalten die erfindungsgemäßen Zusammensetzungen vorzugsweise höhere Mengen solcher Lösungsmittel. Das erfindungsgemäße Mittel wird demnach bevorzugt in einem wässrigen, einem alkoholischen oder in einem wässrig-alkoholischen Medium mit vorzugsweise mindestens 10, besonders bevorzugt mindestens 12 Gewichtsprozent Wasser konfektioniert. Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein.

Als zusätzliche Co-Solventien können organische Lösungsmittel oder ein Gemisch aus Lösungsmitteln mit einem Siedepunkt unter 400°C in einer Menge von 0,1 bis 15 Gewichtsprozent bevorzugt von 1 bis 10 Gewichtsprozent enthalten sein. Besonders geeignet als zusätzliche Co-Solventien sind unverzweigte oder verzweigte Kohlenwasserstoffe wie Pentan, Hexan, Isopentan und cyclische Kohlenwasserstoffe wie Cyclopentan und Cyclohexan. Weitere, besonders bevorzugte wasserlösliche Lösungsmittel sind Glycerin, Ethylenglykol und Propylenglykol in einer Menge bis 30 Gewichtsprozent.

Zusammenfassend sind erfindungsgemäße Kombinationen bevorzugt, bei denen die treibmittelhaltige Antitranspirant-Zusammensetzung bezogen auf ihr Gewicht 10 bis 80 Gew.-%, vorzugsweise 12,5 bis 75 Gew.-%, weiter bevorzugt 15 bis 70 Gew.-%, noch weiter bevorzugt 20 bis 65 Gew.-%, besonders bevorzugt 22,5 bis 62,5 Gew.-% und insbesondere 25 bis 60 Gew.-% mindestens eines protischen Lösungsmittels, ausgewählt aus Ethanol, Wasser und deren Mischungen, enthält.

Die erfindungsgemäßen Zusammensetzungen enthalten als Inhaltsstoff a3) bezogen auf ihr Gewicht 5 bis 60 Gew.-% Treibmittel.

Erfindungsgemäß bevorzugte Treibmittel (Treibgase) sind ausgewählt aus Propan, Propen, n-Butan, iso-Butan, iso-Buten, n-Pentan, Penten, iso-Pentan, iso-Penten, Methan, Ethan, Dimethylether, Stickstoff, Luft, Sauerstoff, Lachgas, Dichlorfluormethan, Chlordifluormethan, Chlorfluormethan, 1,1,2,2-Tetrachlor-1-fluorethan, 1,1,1,2-Tetrachlor-2-fluorethan, 1,2,2-Trichlor-1,1-difluorethan, 1,1,2-Trichlor-1,2-difluorethan, 1,1,1-Trichlor-2,2-difluorethan, 2,2-Dichlor-1,1,1-trifluorethan, 1,2-Dichlor-1,1,2-trifluorethan, 2-Chlor-1,1,1,2-tetrafluorethan, 1-Chlor-1,1,2,2-tetrafluorethan, 1,1,2-Trichlor-2-fluorethan, 1,2-Dichlor-1,2-difluorethan, 1,2-Dichlor-1,1-difluorethan, 1-Chlor-1,2,2-trifluorethan, 2-Chlor-1,1,1-trifluorethan, 1-Chlor-1,1,2-trifluorethan, 1,2-Dichlor-1-fluorethan, 1,1-Dichlor-1-fluorethan, 2-Chlor-1,1-difluorethan, 1-Chlor-1,1-difluorethan, 1-Chlor-2-fluorethan, 1-Chlor-1-fluorethan, 2-Chlor-1,1-difluorethen, 1,1,1,3-Tetrafluorethan, Heptafluoro-n-propan, Perfluorethan, Monochlordifluormethan, 1,1-Difluorethan, und zwar sowohl einzeln als auch in Kombination. Besonders bevorzugt sind Propan, n-Butan, iso-Butan sowie, besonders bevorzugt, Mischungen dieser Treibmittel.

Auch hydrophile Treibgase, wie z. B. Kohlendioxid, können vorteilhaft im Sinne der vorliegenden Erfindung eingesetzt werden, wenn der Anteil an hydrophilen Gasen gering gewählt wird und lipophiles Treibgas (z. B. Propan/Butan) im Überschuss vorliegt. Besonders bevorzugt sind Propan, n-Butan, iso-Butan sowie insbesondere Mischungen dieser Treibgase.

Die Menge der Treibmittel beträgt bevorzugt 20 - 95 Gew.-%, besonders bevorzugt 30 - 90 Gew.-% und außerordentlich bevorzugt 60 - 86 Gew.-%, und weiterhin außerordentlich bevorzugt 75 - 78 Gew.-%, jeweils bezogen auf das Gesamtgewicht der Zubereitung, bestehend aus der erfindungsgemäßen Suspension und dem Treibmittel.

Gemäß einer bevorzugten Ausführungsform enthalten die erfindungsgemäßen Zubereitungen die genannten Kohlenwasserstoffe, Dimethylether oder Mischungen der genannten Kohlenwasserstoffe mit Dimethylether als einziges Treibmittel. Die Erfindung umfaßt aber ausdrücklich auch die Mitverwendung von Treibmittel vom Typ der Fluorchlorkohlenwasserstoffe, insbesondere aber der Fluorkohlenwasserstoffe.

Ganz besonders bevorzugte erfindungsgemäße Kombinationen sind **dadurch gekennzeichnet, daß** die treibmittelhaltige Antitranspirant-Zusammensetzung bezogen auf ihr Gewicht 7,5 bis 57,5 Gew.-%, vorzugsweise 10 bis 55 Gew.-%, weiter bevorzugt 15 bis 52,5 Gew.-%, noch weiter bevorzugt 17,5 bis 55 Gew.-%, besonders bevorzugt 20 bis 50 Gew.-% und insbesondere 25 bis 45 Gew.-% mindestens eines Treibmittels, ausgewählt aus n-Propan, n-Butan, iso-Butan, n-Pentan, Dimethylether und deren Mischungen, enthält.

### Die erfindungsgemäßen Zusammensetzungen können weitere Inhaltsstoffe enthalten:

Die erfindungsgemäßen Zusammensetzungen, die als Emulsion, insbesondere als Öl-in-Wasser-Emulsion oder Polyol-in-Wasser-Emulsion, formuliert sind, enthalten bevorzugt mindestens einen nichtionischen Öl-in-Wasser-Emulgator mit einem HLB-Wert von mehr als 7. Hierbei handelt es sich um dem Fachmann allgemein bekannte Emulgatoren, wie sie beispielsweise in Kirk-Othmer, "Encyclopedia of Chemical Technology", 3. Aufl., 1979, Band 8, Seite 913-916, aufgelistet sind. Für ethoxylierte Produkte wird der HLB-Wert nach der Formel HLB = (100 - L) : 5 berechnet, wobei L der Gewichtsanteil der lipophilen Gruppen, das heißt der Fettalkyl- oder Fettacylgruppen, in den Ethylenoxidaddukten, ausgedrückt in Gewichtsprozent, ist.

Bei der Auswahl erfindungsgemäß geeigneter nichtionischer Öl-in-Wasser-Emulgatoren ist es besonders bevorzugt, ein Gemisch von nichtionischen Öl-in-Wasser-Emulgatoren einzusetzen, um die Stabilität der erfindungsgemäßen Stiftzusammensetzungen optimal einstellen zu können. Die einzelnen Emulgatorkomponenten liefern dabei einen Anteil zum Gesamt-HLB-Wert oder mittleren HLB-Wert des Öl-in-Wasser-Emulgatorgemisches gemäß ihrem Mengenanteil an der Gesamtmenge der Öl-in-Wasser-Emulgatoren. Erfindungsgemäß beträgt der mittlere HLB-Wert des Öl-in-Wasser- Emulgatorgemisches 10 - 19, bevorzugt 12 - 18 und besonders bevorzugt 14 - 17. Um derartige mittlere HLB-Werte zu erzielen, werden bevorzugt Öl-in-Wasser-Emulgatoren aus den HLB-Wertbereichen 10 - 14, 14 - 16 und gegebenenfalls 16 - 19 miteinander kombiniert. Selbstverständlich können die Öl-in-Wasser-Emulgatorgemische auch nichtionische Emulgatoren mit HLB-Werten im Bereich von > 7 - 10 und 19 - 20 enthalten; derartige Emulgatorgemische können erfindungsgemäß ebenfalls bevorzugt sein. Die erfindungsgemäßen Antitranspirant-Zusammensetzungen können aber in einer anderen bevorzugten Ausführungsform auch nur einen einzigen Öl-in-Wasser-Emulgator mit einem HLB-Wert im Bereich von 10 - 19 enthalten.

Bevorzugte erfindungsgemäße Antitranspirant-Zusammensetzungen sind dadurch gekennzeichnet, dass die nichtionischen Öl-in-Wasser-Emulgatoren ausgewählt sind aus ethoxylierten C₈-C₂₄-Alkanolen mit durchschnittlich 10 - 100 Mol Ethylenoxid pro Mol, ethoxylierten C₈-C₂₄-Carbonsäuren mit durchschnittlich 10 - 100 Mol Ethylenoxid pro Mol, Silicon-Copolyolen mit Ethylenoxid-Einheiten oder mit Ethylenoxid- und Propylenoxid-Einheiten, Alkylmono- und -oligoglycosiden mit 8 bis 22 Kohlenstoffatomen im Alkylrest und deren ethoxylierten Analoga, ethoxylierten Sterinen, Partialestern von Polyglycerinen mit 2 bis 10 Glycerineinheiten und mit 1 bis 4 gesättigten oder ungesättigten, linearen oder verzweigten, gegebenenfalls hydroxylierten C₈ - C₃₀-Fettsäureresten verestert, sofern sie einen HLB-Wert von mehr als 7 aufweisen, sowie Mischungen der vorgenannten Substanzen.

Die ethoxylierten C₈-C₂₄-Alkanole haben die Formel R¹O(CH₂CH₂O)ₙH, wobei R¹ steht für einen linearen oder verzweigten Alkyl- und/oder Alkenylrest mit 8 - 24 Kohlenstoffatomen und n, die mittlere Anzahl der Ethylenoxid-Einheiten pro Molekül, für Zahlen von 10 - 100, vorzugsweise 10 - 30 Mol Ethylenoxid an 1 Mol Caprylalkohol, 2-Ethylhexylalkohol, Caprinalkohol, Laurylalkohol, Isotridecylalkohol, Myristylalkohol, Cetylalkohol, Palmitoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen. Auch Addukte von 10 - 100 Mol Ethylenoxid an technische Fettalkohole mit 12 - 18 Kohlenstoffatomen, wie beispielsweise Kokos-, Palm-, Palmkern- oder Talgfettalkohol, sind geeignet.

Die ethoxylierten C₈-C₂₄-Carbonsäuren haben die Formel R¹OCH₂CH₂)ₙOH, wobei R¹ steht für einen linearen oder verzweigten gesättigten oder ungesättigten Acylrest mit 8 -24 Kohlenstoffatomen und n, die mittlere Anzahl der Ethylenoxid-Einheiten pro Molekül, für Zahlen von 10 - 100, vorzugsweise 10 - 30 Mol Ethylenoxid an 1 Mol Caprylsäure, 2-Ethylhexansäure, Caprinsäure, Laurinsäure, Isotridecansäure, Myristinsäure, Cetylsäure, Palmitoleinsäure, Stearinsäure, Isostearinsäure, Ölsäure, Elaidinsäure, Petroselinsäure, Arachinsäure, Gadoleinsäure, Behensäure, Erucasäure und Brassidinsäure sowie deren technische Mischungen. Auch Addukte von 10 - 100 Mol Ethylenoxid an technische Fettsäuren mit 12 - 18 Kohlenstoffatomen, wie Kokos-, Palm-, Palmkern- oder Talgfettsäure, sind geeignet. Besonders bevorzugt sind PEG-50-monostearat, PEG-100-monostearat, PEG-50-monooleat, PEG-100-monooleat, PEG-50-monolaurat und PEG-100-monolaurat.

Besonders bevorzugt eingesetzt werden die C₁₂-C₁₈-Alkanole oder die C₁₂-C₁₈-Carbonsäuren mit jeweils 10 - 30 Einheiten Ethylenoxid pro Molekül sowie Mischungen dieser Substanzen, insbesondere Ceteth-12, Ceteth-20, Ceteth-30, Steareth-12, Steareth-20, Steareth-30, Laureth-12 und Beheneth-20.

Weiterhin werden vorzugsweise C₈ - C₂₂-Alkylmono- und -oligoglycoside eingesetzt. C₈ -C₂₂-Alkylmono- und -oligoglycoside stellen bekannte, handelsübliche Tenside und Emulgatoren dar. Ihre Herstellung erfolgt insbesondere durch Umsetzung von Glucose oder Oligosacchariden mit primären Alkoholen mit 8 - 22 Kohlenstoffatomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis etwa 8, vorzugsweise 1 - 2, geeignet sind. Der Oligomerisierungsgrad ist dabei ein statistischer Mittelwert, dem eine für solche technischen Produkte übliche Homologenverteilung zugrunde liegt. Produkte, die unter dem Warenzeichen Plantacare^{®} erhältlich sind, enthalten eine glucosidisch gebundene C₈-C₁₆-Alkylgruppe an einem Oligoglucosidrest, dessen mittlerer Oligomerisationsgrad bei 1 - 2, insbesondere bei 1,1 - 1,4, liegt. Besonders bevorzugte C₈ - C₂₂-Alkylmono- und -oligoglycoside sind ausgewählt aus Octylglucosid, Decylglucosid, Laurylglucosid, Palmitylglucosid, Isostearylglucosid, Stearylglucosid, Arachidylglucosid und Behenylglucosid sowie Mischungen hiervon. Auch die vom Glucamin abgeleiteten Acylglucamide sind als nicht-ionische Öl-in-Wasser-Emulgatoren geeignet.

Auch ethoxylierte Sterine, insbesondere ethoxylierte Sojasterine, stellen erfindungsgemäß geeignete Öl-in-Wasser-Emulgatoren dar. Der Ethoxylierungsgrad muss größer als 5, bevorzugt mindestens 10 sein, um einen HLB-Wert größer 7 aufzuweisen. Geeignete Handelsprodukte sind z. B. PEG-10 Soy Sterol, PEG-16 Soy Sterol und PEG-25 Soy Sterol.

Weiterhin werden vorzugsweise Partialester von Polyglycerinen mit 2 bis 10 Glycerineinheiten und mit 1 bis 4 gesättigten oder ungesättigten, linearen oder verzweigten, gegebenenfalls hydroxylierten C₈ - C₃₀-Fettsäureresten verestert, eingesetzt, sofern sie einen HLB-Wert von mehr als 7 aufweisen. Besonders bevorzugt sind Diglycerinmonocaprylat, Diglycerinmonocaprat, Diglycerinmonolaurat, Triglycerinmonocaprylat, Triglycerinmonocaprat, Triglycerinmonolaurat, Tetraglycerinmonocaprylat, Tetraglycerinmonocaprat, Tetraglycerinmonolaurat, Pentaglycerinmonocaprylat, Pentaglycerinmonocaprat, Pentaglycerinmonolaurat, Hexaglycerinmonocaprylat, Hexaglycerinmonocaprat, Hexa glycerinmonolaurat, Hexaglycerinmonomyristat, Hexaglycerinmonostearat, Decaglycerinmonocaprylat, Decaglycerinmonocaprat, Decaglycerinmonolaurat, Decaglycerinmonomyristat, Decaglycerinmonoisostearat, Decaglycerinmonostearat, Decaglycerinmonooleat, Decaglycerinmonohydroxystearat, Decaglycerindicaprylat, Decaglycerindicaprat, Decaglycerindilaurat, Decaglycerindimyristat, Decaglycerindiisostearat, Decaglycerindistearat, Decaglycerindioleat, Decaglycerindihydroxystearat, Decaglycerintricaprylat, Decaglycerintricaprat, Decaglycerintrilaurat, Decaglycerintrimyristat, Decaglycerintriisostearat, Decaglycerintristearat, Decaglycerintrioleat und Decaglycerintrihydroxystearat.

Besonders bevorzugte erfindungsgemäße Antitranspirant-Zusammensetzungen sind dadurch gekennzeichnet, dass der nichtionische Öl-in-Wasser-Emulgator in einer Gesamtmenge von 0,5 - 10 Gew.-%, besonders bevorzugt 1 - 4 Gew.-% und außerordentlich bevorzugt 1,5 - 3 Gew.-%, bezogen auf die Gesamtzusammensetzung, enthalten ist.

### Wasser-in-Öl-Emulgatoren

Erfindungsgemäß bevorzugte Zusammensetzungen, die als Emulsion oder Stift formuliert sind, enthalten bevorzugt weiterhin mindestens einen nichtionischen Wasser-in-Öl-Emulgator mit einem HLB-Wert größer 1,0 und kleiner/gleich 7,0, ausgewählt aus den Mono- und Diestern von Ethylenglycol und den Mono-, Di-, Tri- und Tetraestern von Pentaerythrit mit linearen gesättigten Fettsäuren mit 12 - 30, insbesondere 14 - 22 Kohlenstoffatomen, die hydroxyliert sein können, sowie Mischungen hiervon, als Konsistenzgeber und/oder Wasserbinder. Erfindungsgemäß bevorzugt sind die Mono- und Diester. Erfindungsgemäß bevorzugte C₁₂-C₃₀-Fettsäurereste sind ausgewählt aus Laurinsäure-, Myristinsäure-, Palmitinsäure-, Stearinsäure-, Arachinsäure- und Behensäure-Resten; besonders bevorzugt ist der Stearinsäurerest. Erfindungsgemäß besonders bevorzugte nichtionische Wasser-in-Öl-Emulgatoren mit einem HLB-Wert größer 1,0 und kleiner/gleich 7,0 sind ausgewählt aus Pentaerythritylmonostearat, Pentaerythrityldistearat, Pentaerythrityltristearat, Pentaerythrityltetrastearat, Ethylenglycolmonostearat, Ethylenglycoldistearat sowie Mischungen hiervon. Erfindungsgemäß besonders bevorzugte Wasser-in-Öl-Emulgatoren mit einem HLB-Wert größer 1,0 und kleiner/ gleich 7,0 sind zum Beispiel als Handelsprodukte Cutina^{®} PES (INCI: Pentaerythrityl distearate), Cutina^{®} AGS (INCI: Glycol distearate) oder Cutina^{®} EGMS (INCI: Glycol stearate) erhältlich. Diese Handelsprodukte stellen bereits Mischungen aus Mono- und Diestern (bei den Pentaerythritylestern sind auch Tri- und Tetraester enthalten) dar. Erfindungsgemäß kann es bevorzugt sein, nur einen einzigen Wasser-in-Öl-Emulgator einzusetzen. In einer anderen bevorzugten Ausführungsform enthalten die erfindungsgemäßen Zusammensetzungen Mischungen, insbesondere technische Mischungen, von mindestens zwei Wasser-in-Öl-Emulgatoren. Unter einer technischen Mischung wird beispielsweise ein Handelsprodukt wie Cutina^{®} PES verstanden.

Außer den genannten Wasser-in-Öl-Emulgatoren auf Basis der Ethylenglycol- oder Pentaerythritylester kann in einer bevorzugten Ausführungsform auch noch mindestens ein weiterer nichtionischer Wasser-in-Öl-Emulgator mit einem HLB-Wert größer 1,0 und kleiner/gleich 7,0 enthalten sein, dessen Anteil an dem Gesamtgewicht an nichtionischen Wasser-in-Öl-Emulgatoren mit einem HLB-Wert größer 1,0 und kleiner/gleich 7,0 bevorzugt allerdings nicht größer als 80 % sein sollte. In einer besonders bevorzugten Ausführungsform enthalten die erfindungsgemäßen Zusammensetzungen den mindestens einen zusätzlichen Wasser-in-Öl-Emulgator mit einem HLB-Wert größer 1,0 und kleiner/ gleich 7,0 nur in einem Gewichtsanteil von maximal 10 % beziehungsweise sind frei von zusätzlichen Wasser-in-Öl-Emulgatoren. Einige dieser zusätzlichen geeigneten Emulgatoren sind beispielsweise in Kirk-Othmer, "Encyclopedia of Chemical Technology", 3. Aufl., 1979, Band 8, Seite 913, aufgelistet. Für ethoxylierte Addukte lässt sich der HLB-Wert, wie bereits erwähnt, auch berechnen.

Als Wasser-in-Öl-Emulgator bevorzugt geeignet sind:
- lineare gesättigte Alkanole mit 12 - 30 Kohlenstoffatomen, insbesondere mit 16 - 22 Kohlenstoffatomen, insbesondere Cetylalkohol, Stearylalkohol, Arachidylalkohol, Behenylalkohol und Lanolinalkohol oder Gemische dieser Alkohole, wie sie bei der technischen Hydrierung von pflanzlichen und tierischen Fettsäuren erhältlich sind,
- Ester und insbesondere Partialester aus einem Polyol mit 3 - 6 C-Atomen und linearen gesättigten und ungesättigten Fettsäuren mit 12 - 30, insbesondere 14 - 22 C-Atomen, die hydroxyliert sein können. Solche Ester oder Partialester sind z. B. die Mono- und Diester von Glycerin oder die Monoester von Propylenglycol mit linearen gesättigten und ungesättigten C₁₂ - C₃₀-Carbonsäuren, die hydroxyliert sein können, insbesondere diejenigen mit Palmitin- und Stearinsäure, die Sorbitanmono-, -di- oder -triester von linearen gesättigten und ungesättigten C₁₂ - C₃₀-Carbonsäuren, die hydroxyliert sein können, insbesondere diejenigen von Myristinsäure, Palmitinsäure, Stearinsäure oder von Mischungen dieser Fettsäuren und die Methylglucosemono- und -diester von linearen gesättigten und ungesättigten C₁₂ - C₃₀-Carbonsäuren, die hydroxyliert sein können;
- Sterine, also Steroide, die am C3-Atom des Steroid-Gerüstes eine Hydroxylgruppe tragen und sowohl aus tierischem Gewebe (Zoosterine, z. B. Cholesterin, Lanosterin) wie auch aus Pflanzen (Phytosterine, z. B. Ergosterin, Stigmasterin, Sitosterin) und aus Pilzen und Hefen (Mykosterine) isoliert werden und die niedrig ethoxyliert (1 - 5 EO) sein können;
- Alkanole und Carbonsäuren mit jeweils 8 - 24 C-Atomen, insbesondere mit 16 - 22 C-Atomen, in der Alkylgruppe und 1 - 4 Ethylenoxid-Einheiten pro Molekül, die einen HLB-Wert größer 1,0 und kleiner/gleich 7,0 aufweisen,
- Glycerinmonoether gesättigter und/oder ungesättigter, verzweigter und/oder unverzweigter Alkohole einer Kettenlänge von 8 - 30, insbesondere 12 - 18 Kohlenstoffatomen.
- Partialester von Polyglycerinen mit n = 2 bis 10 Glycerineinheiten und mit 1 bis 5 gesättigten oder ungesättigten, linearen oder verzweigten, gegebenenfalls hydroxylierten C₈ - C₃₀-Fettsäureresten verestert, sofern sie einen HLB-Wert von kleiner/gleich 7 aufweisen,
- sowie Mischungen der vorgenannten Substanzen.

Erfindungsgemäß kann es bevorzugt sein, nur einen einzigen zusätzlichen Wasser-in-Öl-Emulgator einzusetzen. In einer anderen bevorzugten Ausführungsform enthalten die erfindungsgemäßen Zusammensetzungen Mischungen, insbesondere technische Mischungen, von mindestens zwei zusätzlichen Wasser-in-Öl-Emulgatoren. Unter einer technischen Mischung wird beispielsweise ein Handelsprodukt wie Cutina^{®} GMS verstanden, das eine Mischung aus Glycerylmonostearat und Glyceryldistearat darstellt.

Besonders vorteilhaft einsetzbare zusätzliche Wasser-in-Öl-Emulgatoren sind Stearylalkohol, Cetylalkohol, Glycerylmonostearat, insbesondere in Form der Handelsprodukte Cutina^{®} GMS und Cutina^{®} MD (ex Cognis), Glyceryldistearat, Glycerylmonocaprinat, Glycerylmonocaprylat, Glycerylmonolaurat, Glycerylmonomyristat, Glycerylmonopalmitat, Glycerylmonohydroxystearat, Glycerylmonooleat, Glycerylmonolanolat, Glyceryldimyristat, Glyceryldipalmitat, Glyceryldioleat, Propylenglycolmonostearat, Propylenglycolmonolaurat, Sorbitanmonocaprylat, Sorbitanmonolaurat, Sorbitanmonomyristat, Sorbitanmonopalmitat, Sorbitanmonostearat, Sorbitansesquistearat, Sorbitandistearat, Sorbitandioleat, Sorbitansesquioleat, Saccharosedistearat, Arachidylalkohol, Behenylalkohol, Polyethylenglycol(2)stearylether (Steareth-2), Steareth-5, Oleth-2, Diglycerinmonostearat, Diglycerinmonoisostearat, Diglycerinmonooleat, Diglycerindihydroxystearat, Diglycerindistearat, Diglycerindioleat, Triglycerindistearat, Tetraglycerinmonostearat, Tetraglycerindistearat, Tetraglycerintristearat, Decaglycerinpentastearat, Decaglycerinpentahydroxystearat, Decaglycerinpentaisostearat, Decaglycerinpentaoleat, Soy Sterol, PEG-1 Soy Sterol, PEG-5 Soy Sterol, PEG-2-monolaurat und PEG-2-monostearat.

Besonders bevorzugte erfindungsgemäße Antitranspirant-Zusammensetzungen sind dadurch gekennzeichnet, dass mindestens ein Wasser-in-Öl-Emulgator in einer Gesamtmenge von 0,1 - 15 Gew.-%, bevorzugt 0,5 - 8,0 Gew.-%, und besonders bevorzugt 1 - 4 Gew.-%, bezogen auf die Gesamtzusammensetzung, enthalten sind. Weiterhin können auch Mengen von 2 - 3 Gew.-%, bezogen auf das Gesamtgewicht der Zusammensetzung, erfindungsgemäß außerordentlich bevorzugt sein.

In der nachfolgenden Tabelle sind verschiedene Öl-in-Wasser-Emulgatoren und Wasser-in-Öl-Emulgatoren und ihre HLB-Werte zusammengestellt. Die HLB-Werte können aber auch nach Griffin berechnet werden, wie es beispielsweise im RÖMPP Chemie Lexikon, insbesondere in der Online-Version von November 2003, und den dort unter dem Stichwort "HLB-System" zitierten Handbüchern von Fiedler, Kirk-Othmer und Janistyn dargestellt beziehungsweise tabelliert ist. Sofern es in der Literatur unterschiedliche Angaben zum HLB-Wert einer Substanz gibt, sollte derjenige HLB-Wert für die erfindungsgemäße Lehre genutzt werden, der dem nach Griffin berechneten Wert am nächsten kommt. Falls sich auf diese Art und Weise kein eindeutiger HLB-Wert ermitteln lässt, ist der HLB-Wert, den der Hersteller des Emulgators angibt, für die erfindungsgemäße Lehre zu nutzen. Falls auch dies nicht möglich ist, ist der HLB-Wert experimentell zu ermitteln.

| HLB-Wert | Chemische Bezeichnung |
|---|---|
| (aus H. Janistyn, Handbuch der Kosmetika und Riechstoffe, Hüthig-Verlag Heidelberg, 3. Auflage, 1978, Band 1, Seite 470 und Band 3, Seiten 68 - 78)) | |
| 1 | Triglyceride gesättigter Fettsäuren |
| | Glyceryltrioleat |
| 1,5 | Ethylenglycoldistearat |
| 1,6 | Pur-Cellinöl |
| 1,8 | Sorbitantrioleat |
| | Glycerindioleat |
| 2,1 | Sorbitantristearat |
| 2,4 | Propylenglycollactostearat |
| 2,7 | Glycerinmonooleat |
| | Sorbitdioleat |
| 2,8 | Glycerinmonostearat |
| | Propylenglycolmono-/distearat, nicht selbstemulgierend |
| 2,9 | Ethylenglycolmonostearat |
| 3,0 | Decaglycerindecaoleat |
| | Decaglycerindecastearat |
| | Generol 122 (Rapeseed Sterols) |
| | Sucrosedistearat |
| 3,1 | Decaglycerindecaoleat |
| | Glycerylmonoricinoleat |
| | Pentaerythritylmonostearat |
| | Pentaerythritylsesquioleat |
| 3,2 | Ethylenglycolmonodistearat, nicht selbstemulgierend |
| | Glycolstearat |
| 3,3 | Glycerinmonolaurat |
| 3,4 | Propylenglycolmonostearat |
| 3,5 | Ethylenglycolmonostearat |
| | Pentaerythritylmonooleat |
| | Polyethylenglycol(100)monooleat |
| 3,6 | Glycerinmono-/dioleat, nicht selbstemulgierend |
| | Monoethoxylaurylether |
| 3,7 | Sorbitansesquioleat (Dehymuls SSO) |
| 3,8 | Glycerinmonodistearat, nicht selbstemulgierend |
| | Polyethylenglycol(100)monostearat |
| | Diglycerinsesquioleat |
| | N,N-Dimethylcaproamid |
| | Pentaerythritmonotallat |
| | Propylenglycolmonolaurat |
| 4,0 | Decaglycerinoctaoleat |
| 4,3 | Sorbitanmonooleat (Dehymuls SMO) |
| | Diethylenglycolmonostearat |
| 4,4 | 1.2-Propylenglycolmonodistearat, selbstemulgierend |
| 4,5 | Glycerinmonostearatpalmitat (90 %), nicht selbstemulgierend |
| | Propylenglycolmonolaurat |
| 4,7 | Sorbitanmonostearat (Dehymuls SMS) |
| | Diethylenglycolmonooleat |
| 4,8 | Pentaerythritmonolaurat |
| 4,9 | Polyoxyethylen(2)oleylalkohol (Polyoxyethylen(2)oleylether) |
| | Polyoxyethylen(2)stearylalkohol (Polyoxyethylen(2)stearylether) |
| 5,0 | Ethylenglycolmonodistearat |
| | Generol 122 E 5 (PEG-5 Soy Sterol) |
| | Polyethylenglycol(100)monoricinoleat |
| | Polyethylenglycol(200)distearat |
| | Polyglyceryl-3-isostearate (z. B. Isolan Gl 34 von Tego) |
| 5,9 | Polyethylenglycol(200)dilaurat |
| 6,0 | Decaglycerintetraoleat |
| | Polyethylenglycol(100)monolaurat |
| | Polyethylenglycol(200)dioleat |
| 6,1 | Diethylenglycolmonolaurat (Diglycollaurat) |
| 6,3 | Polyethylenglycol(300)dilaurat |
| 6,4 | Glycerinmonoricinoleat |
| | Glycerinsorbitanmonolaurat |
| 6,5 | Diethylenglycolmonolaurat |
| | Natriumstearoyl-2-lactylat |
| 6,7 | Sorbitanmonopalmitat |
| 6,8 | Glycerinmonococoat |
| | Glycerinmonolaurat |
| 7,0 | Polyoxyethylen(2)C₁₀-C₁₄-fettalkoholether, Laureth-2 (Dehydol LS 2) |
| | Saccharosedistearat |
| 7,2 | Polyethylenglycol(400)dioleat |
| | Saccharosedioleat |
| 7,4 | Polyethylenglycol(100)monolaurat |
| | Saccharosedipalmitat |
| 7,5 | Saccharosedipalmitat |
| 7,6 | Glycerinsorbitanlaurat |
| 7,8 | Polyethylenglycol(400)distearat |
| 7,9 | Polyethylenglycol(200)monostearat |
| | Polyoxyethylen(3)tridecylalkohol |
| 8-8,2 | Polyethylenglycol(400)distearat |
| 8,0 | Polyoxyethylen(3)C₁₀-C₁₄-fettalkoholether, Laureth-3 (Dehydol LS 3) |
| | N.N-Dimethyllauramid |
| | Natriumlauroyllactylat, Natriumlauroyl-2-lactylat |
| | Polyethylenglycol(200)monooleat |
| | Polyethylenglycol(220)monotallat |
| | Polyethylenglycol(1500)dioleat |
| | Polyoxyethylen(4)oleylalkohol |
| | Polyoxyethylen(4)stearylcetylether |
| 8,2 | Triglycerinmonooleat |
| 8,3 | Diethylenglycolmonolaurat |
| 8,4 | Polyoxyethylen(4)cetylether |
| | Polyoxyethylenglycol(400)dioleat |
| 8,5 | Natriumcaproyllactylat |
| | Polyethylenglycol(200)monostearat |
| | Sorbitanmonooleat |
| 8,6 | Sorbitanmonolaurat (Dehymuls SML) |
| | Polyethylenglycol(200)monolaurat |
| 8,8 | Polyoxyethylen(4)myristylether |
| | Polyethylenglycol (400)dioleat |
| 8,9 | Nonylphenol, polyoxyethyliert mit 4 Mol EO |
| 9,0 | Oleth-5 (z. B. Eumulgin O 5) |
| 9,2 - 9,7 | Polyoxyethylen(4)laurylalkohol (je nach Handelsprodukt, z. B. Brij 30, Dehydol LS 4) |
| 9,3 | Polyoxyethylen(4)tridecylalkohol |
| 9,6 | Polyoxyethylen(4)sorbitanmonostearat |
| 9,8 | Polyethylenglycol(200)monolaurat |
| 10-11 | Polyethylenglycol(400)monooleat |
| 10,0 | Didodecyldimethylammoniumchlorid |
| 10,0 | Polyethylenglycol(200)monolaurat |
| | Polyethylenglycol(400)dilaurat |
| | Polyethylenglycol(600)dioleat |
| | Polyoxyethylen(4)sorbitanmonostearat |
| | Polyoxyethylen(5)sorbitanmonooleat |
| 10,2 | Polyoxyethylen 40)sorbitol hexaoleat |
| 10,4 - 10,6 | Polyoxyethylenglycol(600)distearat |
| 10,5 | Polyoxyethylen(20)sorbitantristearat |
| 10,6 | Saccharosemonostearat |
| 10,7 | Saccharosemonooleat |
| 11 - 11,4 | Polyethylenglycol(400)monooleat |
| 11,0 | Polyethylenglycol(350)monostearat |
| | Polyethylenglycol(400)monotallat |
| | Polyoxyethylenglycol(7)monostearat |
| | Polyoxyethylenglycol(8)monooleat |
| | Polyoxyethylen(20)sorbitantrioleat |
| | Polyoxyethylen(6)tridecylalkohol |
| 11,1 | Polyethylenglycol(400)monostearat |
| 11,2 | Polyoxyethylen(9)monostearat |
| | Saccharosemonooleat |
| | Saccharosemonostearat |
| 11,4 | Polyoxyethylen(50)sorbitol hexaoleat |
| | Saccharosemonotallat |
| | Saccharosestearatpalmitat |
| 11,6 | Polyoxyethylenglycol(400)monoricinoleat |
| 11,7 | Saccharosemonomyristat |
| | Saccharosemonopalmitat |
| 12,0 | PEG-10 Soy Sterol (z. B. Generol 122 E 10) |
| | Triethanolaminoleat |
| 12,2-12,3 | Nonylphenol, ethoxyliert mit 8 Mol EO |
| 12,2 | Saccharosemonomyristat |
| 12,4 | Saccharosemonolaurat |
| | Polyoxyethylen(10)oleylalkohol, Polyoxyethylen(10)oleylether |
| | Polyoxyethylen(10)stearylalkohol, Polyoxyethylen(10)stearylether |
| 12,5 | Polyoxyethylen(10)stearylcetylether |
| 12,7 | Polyoxyethylen(8)tridecylalkohol |
| 12,8 | Polyoxyethylenglycol(400)monolaurat |
| | Saccharosemonococoat |
| 12,9 | Polyoxyethylen(1 0)cetylether |
| 13 | Glycerinmonostearat, ethoxyliert (20 Mol EO) |
| 13,0 | Eumulgin O 10 |
| | Eumulgin 286 |
| | Eumulgin B 1 (Ceteareth-12) |
| 13,0 | C12-Fettamine, ethoxyliert (5 Mol EO) |
| 13,1 | Nonylphenol, ethoxyliert (9,5 Mol EO) |
| 13,2 | Polyethylenglycol(600)monostearat |
| | Polyoxyethylen(16)tallöl |
| 13,3 | Polyoxyethylen(4)sorbitanmonolaurat |
| 13,5 | Nonylphenol, ethoxyliert (10,5 Mol EO) |
| | Polyethylenglycol(600)monooleat |
| 13,7 | Polyoxyethylen(10)tridecylalkohol |
| | Polyethylenglycol(660)monotallat |
| | Polyethylenglycol(1500)monostearat |
| | Polyoxyethylenglycol(1500)dioleat |
| 13,9 | Polyethylenglycol(400)monococoat |
| | Polyoxyethylen(9)monolaurat |
| 14-16 | Eumulgin HRE 40 (Ricinusöl, mit 40 EO ethoxyliert und hydriert) |
| 14,0 | Polyoxyethylen(12)laurylether |
| | Polyoxyethylen(12)tridecylalkohol |
| 14,2 | Polyoxyethylen(1 5)stearylalkohol |
| 14,3 | Polyoxyethylen(15)stearylcetylether |
| 14,4 | Gemisch aus C12-C15-Fettalkoholen mit 12 Mol EO |
| 14,5 | Polyoxyethylen(12)laurylalkohol |
| 14,8 | Polyoxyethylenglycol(600)monolaurat |
| 14,9 - 15,2 | Sorbitanmonostearat, mit 20 EO ethoxyliert (z. B. Eumulgin SMS 20) |
| 15 - 15,9 | Sorbitanmonooleat, mit 20 EO ethoxyliert (z. B. Eumulgin SMO 20) |
| 15,0 | PEG-20 Glyceryl stearate (z. B. Cutina E 24) |
| | PEG-40 Castor Oil (z. B. Eumulgin RO 40) |
| | Decylglucosid (Oramix NS 10) |
| | Dodecylglucosid (Plantaren APG 600) |
| | Dodecyltrimethylammoniumchlorid |
| | Nonylphenol, ethoxyliert mit 15 Mol EO |
| | Polyethyleng lycol(1000)monostearat |
| | Polyoxyethylen(600)monooleat |
| 15-17 | Eumulgin HRE 60 (Ricinusöl, mit 60 EO ethoxyliert und hydriert) |
| 15,3 | C12-Fettamine, polyoxyethyliert mit 12 Mol EO |
| | Polyoxyethylen(20)oleylalkohol, Polyoxyethylen(20)oleylether |
| 15,4 | Polyoxyethylen(20)stearylcetylether (z. B. Eumulgin B 2 (Ceteareth-20)) |
| 15,5 | Polyoxyethylen(20)stearylalkohol |
| 15,6 | Polyoxyethylenglycol(1000)monostearat |
| | Polyoxyethylen(20)sorbitanmonopalmitat |
| 15,7 | Polyoxyethylen(20)cetylether |
| 15,9 | Dinatriumtriethanolamindistearylheptaglycolethersulfosuccinat |
| 16,0 | Nonylphenol ethoxyliert mit 20 Mol EO |
| | Polyoxyethylen(25)propylenglycolstearat |
| 16 - 16,8 | Polyoxyethylen(30)monostearat |
| 16,3-16,9 | Polyoxyethylen(40)monostearat |
| 16,5 - 16,7 | Polyoxyethylen(20)sorbitanmonolaurat (z. B. Eumulgin SML 20) |
| 16,6 | Polyoxyethylen(20)sorbit |
| 16,7 | C18-Fettamine, polyoxyethyliert mit 5 Mol EO |
| | Polyoxyethylen(23)laurylalkohol |
| 17,0 | Ceteareth-30, z. B. Eumulgin B 3 |
| | Octylglucosid (Triton CG 110) |
| | Polyoxyethylen(30)glycerylmonolaurat |
| 17,1 | Nonylphenol, ethoxyliert mit 30 Mol EO |
| 17,4 | Polyoxyethylen(40)stearylalkohol |

Weitere bevorzugte erfindungsgemäße Zusammensetzungen sind dadurch gekennzeichnet, dass der Gesamtgehalt an nichtionischen und ionischen Emulgatoren und/oder Tensiden mit einem HLB-Wert über 8 maximal 20 Gew.-%, bevorzugt maximal 15 Gew.-%, besonders bevorzugt maximal 10 Gew.-%, besonders bevorzugt maximal 7 Gew.-%, weiterhin besonders bevorzugt maximal 4 Gew.-% und außerordentlich bevorzugt maximal 3 Gew.-%, jeweils bezogen auf die gesamte erfindungsgemäße Zusammensetzung, beträgt.

### Öle

Bevorzugte erfindungsgemäße Zusammensetzungen, die als Emulsion bzw. Suspension vorliegen, enthalten bevorzugt weiterhin mindestens ein bei 20 ° C flüssiges Öl, das keine Duftstoffkomponente und kein ätherisches Öl darstellt. Erfindungsgemäß bevorzugte Öle sind ausgewählt aus verzweigten gesättigten oder ungesättigten Fettalkoholen mit 6 - 30 Kohlenstoffatomen. Diese Alkohole werden häufig auch als Guerbet-Alkohole bezeichnet, da sie nach der Guerbet-Reaktion erhältlich sind. Bevorzugte Alkoholöle sind Hexyldecanol (Eutanol^{®} G 16, Guerbitol^{®} T 16), Octyldodecanol (Eutanol^{®} G, Guerbitol^{®} 20), 2-Ethylhexylalkohol und die Handelsprodukte Guerbitol^{®} 18, Isofol^{®} 12, Isofol^{®} 16, Isofol^{®} 24, Isofol^{®} 36, Isocarb^{®} 12, Isocarb^{®} 16 oder Isocarb^{®} 24. Weitere bevorzugte Ölkomponenten sind Mischungen aus Guerbetalkoholen und Guerbetalkoholestern, z.B. das Handelsprodukt Cetiol^{®} PGL (Hexyldecanol und Hexyldecyllaurat).

Weitere erfindungsgemäß bevorzugte Öle sind ausgewählt aus den Triglyceriden von linearen oder verzweigten, gesättigten oder ungesättigten, gegebenenfalls hydroxylierten C₈₋₃₀-Fettsäuren. Besonders geeignet kann die Verwendung natürlicher Öle, z.B. Sojaöl, Baumwollsaatöl, Sonnenblumenöl, Palmöl, Palmkernöl, Leinöl, Mandelöl, Rizinusöl, Maisöl, Olivenöl, Rapsöl, Sesamöl, Distelöl, Weizenkeimöl, Pfirsichkernöl und die flüssigen Anteile des Kokosöls und dergleichen sein. Geeignet sind aber auch synthetische Triglyceridöle, insbesondere Capric/Caprylic Triglycerides, z. B. die Handelsprodukte Myritol^{®} 318, Myritol^{®} 331 (Cognis) oder Miglyol^{®} 812 (Hüls) mit unverzweigten Fettsäureresten sowie Glyceryltriisostearin und die Handelsprodukte Estol^{®} GTEH 3609 (Uniqema) oder Myritol^{®} GTEH (Cognis) mit verzweigten Fettsäureresten.

Weitere erfindungsgemäß besonders bevorzugte Öle sind ausgewählt aus den Dicarbonsäureestern von linearen oder verzweigten C₂-C₁₀-Alkanolen, insbesondere Diisopropyladipat, Di-n-butyladipat, Di-(2-ethylhexyl)adipat, Dioctyladipat, Diethyl-/Di-n-butyl/Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat.

Weitere erfindungsgemäß besonders bevorzugte Öle sind ausgewählt aus den Anlagerungsprodukten von 1 bis 5 Propylenoxid-Einheiten an ein- oder mehrwertige C₈₋₂₂-Alkanole wie Octanol, Decanol, Decandiol, Laurylalkohol, Myristylalkohol und Stearylalkohol, z. B. PPG-2-Myristylether und PPG-3-Myristylether (Witconol^{®} APM).

Weitere erfindungsgemäß bevorzugte Ölkomponenten sind ausgewählt aus den Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können. Dazu zählen Hexyldecylstearat (Eutanol^{®} G 16 S), Hexyldecyllaurat, Isodecylneopentanoat, Isononylisononanoat, 2-Ethylhexylpalmitat (Cegesoft^{®} C 24) und 2-Ethylhexylstearat (Cetiol^{®} 868). Ebenfalls bedingt geeignet sind Isopropylmyristat, Isopropylpalmitat, Isopropylstearat, Isopropylisostearat, Isopropyloleat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Butylstearat, n-Hexyllaurat, n-Decyloleat, Oleyloleat, Oleylerucat, Erucyloleat, Erucylerucat, Ethylenglycoldioleat und -dipalmitat.

Weitere erfindungsgemäß bevorzugte Ölkomponenten sind ausgewählt aus den Anlagerungsprodukten von mindestens 6 Ethylenoxid und/oder Propylenoxid-Einheiten an ein- oder mehrwertige C₃₋₂₂-Alkanole wie Butanol, Butandiol, Myristylalkohol und Stearylalkohol, z. B. PPG-14-Butylether (Ucon Fluid^{®} AP), PPG-9-Butylether (Breox^{®} B25), PPG-10-Butandiol (Macol^{®} 57) und PPG-15-Stearylether (Arlamol^{®} E).

Weitere erfindungsgemäß bevorzugte Ölkomponenten sind ausgewählt aus den C₈-C₂₂-Fettalkoholestern einwertiger oder mehrwertiger C₂-C₇-Hydroxycarbonsäuren, insbesondere die Ester der Glycolsäure, Milchsäure, Äpfelsäure, Weinsäure, Citronensäure und Salicylsäure. Solche Ester auf Basis von linearen C_{14/15}-Alkanolen, z. B. C₁₂-C₁₅-Alkyllactat, und von in 2-Position verzweigten C_{12/13}-Alkanolen sind unter dem Warenzeichen Cosmacol^{®} von der Firma Nordmann, Rassmann GmbH & Co, Hamburg, zu beziehen, insbesondere die Handelsprodukte Cosmacol^{®} ESI, Cosmacol^{®} EMI und Cosmacol^{®} ETI.

Weitere erfindungsgemäß bevorzugte Ölkomponenten sind ausgewählt aus den symmetrischen, unsymmetrischen oder cyclischen Estern der Kohlensäure mit Fettalkoholen, z. B. Glycerincarbonat, Dicaprylylcarbonat (Cetiol^{®} CC) oder die Ester der DE 197 56 454 A1.

Weitere erfindungsgemäß bevorzugte Ölkomponenten sind ausgewählt aus den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen.

Es kann erfindungsgemäß außerordentlich bevorzugt sein, Mischungen der vorgenannten Öle einzusetzen.

### Weitere erfindungsgemäß bevorzugte Ölkomponenten sind ausgewählt aus Siliconölen und Kohlenwasserstoffölen.

Erfindungsgemäß bevorzugte Siliconöle sind ausgewählt aus Dialkyl- und Alkylarylsiloxanen, wie beispielsweise Cyclopentasiloxan, Cyclohexasiloxan, Dimethylpolysiloxan und Methylphenylpolysiloxan, aber auch Hexamethyldisiloxan, Octamethyltrisiloxan und Decamethyltetrasiloxan zählen.

Weitere erfindungsgemäß bevorzugte Siliconöle sind ausgewählt aus flüchtigen Siliconölen, die cyclisch sein können, wie z. B. Octamethylcyclotetrasiloxan, Decamethylcyclopentasiloxan und Dodecamethylcyclohexasiloxan sowie Mischungen hiervon, wie sie z. B. in den Handelsprodukten DC 244, 245, 344 und 345 von Dow Corning enthalten sind, oder linear, z. B. Hexamethyldisiloxan (L₂), Octamethyltrisiloxan (L₃), Decamethyltetrasiloxan (L₄), beliebige Zweier- und Dreiermischungen aus L₂, L₃ und/oder L₄, wie sie z. B. in den Handelsprodukten DC 2-1184, Dow Corning^{®} 200 (0, 65 cSt) und Dow Corning^{®} 200 (1,5 cSt) von Dow Corning enthalten sind.

Weitere erfindungsgemäß bevorzugte Siliconöle sind ausgewählt aus nichtflüchtigen höhermolekularen linearen Dimethylpolysiloxanen, im Handel erhältlich z. B. unter der Bezeichnung Dow Corning^{®} 190, Dow Corning^{®} 200 Fluid mit Viskositäten im Bereich von 5 - 100 cSt, bevorzugt 5 - 50 cSt oder auch 5 - 10 cSt, und Baysilon^{®} 350 M.

Erfindungsgemäß bevorzugte natürliche und synthetische Kohlenwasserstoffe sind ausgewählt aus Paraffinölen, Isohexadecan, Isoeicosan, Polyisobutenen und Polydecenen, die beispielsweise unter der Bezeichnung Emery^{®} 3004, 3006, 3010 oder unter der Bezeichnung Ethylflo^{®} von Albemarle oder Nexbase^{®} 2004G von Nestle erhältlich sind, sowie 1,3-Di-(2-ethylhexyl)-cyclohexan (Cetiol^{®}S).

Besonders bevorzugte erfindungsgemäße Antitranspirant-Zusammensetzungen sind dadurch gekennzeichnet, dass das/die bei 20 °C flüssige/n Öl/e in einer Gesamtmenge von 0,1 - 80 Gew.-%, bevorzugt 2 - 20 Gew.-%, besonders bevorzugt 3 - 15 Gew.-%, jeweils bezogen auf das Gesamtgewicht der Zusammensetzung, enthalten ist/sind.

In einer weiteren bevorzugten Ausführungsform der Erfindung weist ein Anteil der Ölkomponenten von mindestens 80 Gew.-% einen Brechungsindex n_{D} von 1,39 - 1,51 auf. Besonders bevorzugt ist es, wenn 5 - 40 - 50 Gew.-%, außerordentlich bevorzugt 10 - 12 - 25 - 30 Gew.-% der Ölkomponenten einen Brechungsindex n_{D} von 1,43 - 1,51, bevorzugt 1,44 - 1,49, besonders bevorzugt 1,45 - 1,47 - 1,485, bei 20 °C (gemessen bei λ = 589 nm) aufweisen.

Erfindungsgemäß besonders bevorzugte Zusammensetzungen, die als Wasser-in-Öl-Emulsion konfektioniert sind, enthalten bevorzugt weiterhin mindestens einen Wasser-in-Öl-Emulgator. Der mindestens eine Wasser-in-Öl-Emulgator ist bevorzugt in einer Menge von 0,5 - 5 Gew.-%, besonders bevorzugt 1,0 - 1,5 - 2,5 Gew.-%, jeweils bezogen auf das Gesamtgewicht der Emulsion, enthalten.

Eine erfindungsgemäß besonders bevorzugte Gruppe von Wasser-in-Öl-Emulgatoren sind die Poly-(C₂-C₃)alkylenglycol-modifizierten Silicone, deren frühere INCI-Bezeichnung Dimethicone Copolyol lautete, mit den aktuellen INCI-Bezeichnungen PEG-x Dimethicone (mit x = 2 - 20, bevorzugt 3 - 17, besonders bevorzugt 11 - 12), Bis-PEG-y Dimethicone (mit y = 3 - 25, bevorzugt 4 - 20), PEG/PPG a/b Dimethicone (wobei a und b unabhängig voneinander für Zahlen von 2 - 30, bevorzugt 3 - 30 und besonders bevorzugt 12 - 20, insbesondere 14 - 18, stehen), Bis-PEG/PPGc/d Dimethicone (wobei c und d unabhängig voneinander für Zahlen von 10 - 25, bevorzugt 14 - 20 und besonders bevorzugt 14 - 16, stehen) und Bis-PEG/PPG-e/f PEG/PPG g/h Dimethicone (wobei e, f, g und h unabhängig voneinander für Zahlen von 10 - 20, bevorzugt 14 - 18 und besonders bevorzugt 16, stehen). Besonders bevorzugt sind PEG/PPG-18/18 Dimethicone, das in einer 1:9-Mischung mit Cyclomethicone als DC 3225 C bzw. DC 5225 C im Handel erhältlich ist, PEG/PPG-4/12 Dimethicone, das unter der Bezeichnung Abil B 8852 erhältlich ist, sowie Bis-PEG/PPG-14/14 Dimethicone, das in einer Mischung mit Cyclomethicone als Abil EM 97 (Goldschmidt) im Handel erhältlich ist, Bis-PEG/PPG-20/20 Dimethicone, das unter der Bezeichnung Abil B 8832 erhältlich ist, PEG/PPG-5/3 Trisiloxane (Silsoft 305), sowie PEG/PPG-20/23 Dimethicone (Silsoft 430 und Silsoft 440).

Weitere erfindungsgemäß bevorzugte W/O-Emulgatoren sind Poly-(C₂-C₃)alkylenglycol-modifizierte Silicone, die mit C₄-C₁₈-Alkylgruppen hydrophob modifiziert sind, besonders bevorzugt Cetyl PEG/PPG-10/1 Dimethicone (früher: Cetyl Dimethicone Copolyol, erhältlich als Abil EM 90 oder in einer Mischung aus Polyglyceryl-4-isostearat, Cetyl PEG/PPG-10/1 Dimethicone und Hexyllaurat unter der Handelsbezeichnung Abil WE 09), weiterhin Alkyl Methicone Copolyole und Alkyl Dimethicone Ethoxy Glucoside.

Erfindungsgemäß besonders bevorzugte Zusammensetzungen enthalten weiterhin bevorzugt mindestens einen hautkühlenden Wirkstoff. Erfindungsgemäß geeignete hautkühlende Wirkstoffe sind beispielsweise Menthol, Isopulegol sowie Mentholderivate, z. B. Menthyllactat, Menthylglycolat, Menthylpyrrolidoncarbonsäure, Menthylmethylether, Menthoxypropandiol, Menthonglycerinacetal (9-Methyl-6-(1-methylethyl)-1,4- dioxaspiro (4.5)decan-2-methanol), Monomenthylsuccinat und 2-Hydroxymethyl-3,5,5-trimethylcyclohexanol. Als hautkühlende Wirkstoffe bevorzugt sind Menthol, Isopulegol, Menthyllactat, Menthoxypropandiol und Menthylpyrrolidoncarbonsäure sowie Mischungen dieser Substanzen, insbesondere Mischungen von Menthol und Menthyllactat, Menthol, Mentholglycolat und Menthyllactat, Menthol und Menthoxypropandiol oder Menthol und Isopulegol.

Erfindungsgemäß besonders bevorzugt ist, dass mindestens ein hautkühlender Wirkstoff in einer Gesamtmenge von 0,01 - 1 Gew.%, besonders bevorzugt 0,02 - 0,5 Gew.% und außerordentlich bevorzugt 0,05 - 0,2 Gew.%, jeweils bezogen auf das Gesamtgewicht der Zusammensetzung, enthalten ist.

### Polyole

Bevorzugte erfindungsgemäße Zusammensetzungen enthalten weiterhin mindestens ein wasserlösliches mehrwertiges C₂ - C₉-Alkanol mit 2 - 6 Hydroxylgruppen und/oder mindestens ein wasserlösliches Polyethylenglycol mit 3 - 20 Ethylenoxid-Einheiten sowie Mischungen hiervon. Bevorzugt sind diese Komponenten ausgewählt aus 1,2-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen wie 1,2-Pentandiol und 1,5-Pentandiol, Hexandiolen wie 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit sowie Mischungen der vorgenannten Substanzen. Geeignete wasserlösliche Polyethylenglycole sind ausgewählt aus PEG-3, PEG-4, PEG-6, PEG-7, PEG-8, PEG-9, PEG-10, PEG-12, PEG-14, PEG-16, PEG-18 und PEG-20 sowie Mischungen hiervon, wobei PEG-3 bis PEG-8 bevorzugt sind.

Bevorzugte erfindungsgemäße Antitranspirant-Zusammensetzungen sind dadurch gekennzeichnet, dass das mindestens eine wasserlösliche mehrwertige C₂ - C₉-Alkanol mit 2 - 6 Hydroxylgruppen und/oder mindestens eine wasserlösliche Polyethylenglycol mit 3 - 20 Ethylenoxid-Einheiten ausgewählt ist aus 1,2-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen wie 1,2-Pentandiol und 1,5-Pentandiol, Hexandiolen wie 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit sowie Mischungen der vorgenannten Substanzen.

Besonders bevorzugte erfindungsgemäße Antitranspirant-Zusammensetzungen sind dadurch gekennzeichnet, dass das mindestens eine wasserlösliche mehrwertige C₂ - C₉-Alkanol mit 2 - 6 Hydroxylgruppen und/oder mindestens eine wasserlösliche Polyethylenglycol mit 3 - 20 Ethylenoxid-Einheiten insgesamt in Mengen von 3 - 30 Gew.-%, bevorzugt 8 - 25 Gew.-%, besonders bevorzugt 10 - 18 Gew.-%, jeweils bezogen auf die Gesamtzusammensetzung, enthalten ist.

Besonders bevorzugte erfindungsgemäße Antitranspirant-Zusammensetzungen sind dadurch gekennzeichnet, dass weiterhin mindestens eine Duftstoffkomponente enthalten ist.

Als Duftstoffkomponente können Parfüme, Parfümöle oder Parfümölbestandteile eingesetzt werden. Parfümöle bzw. Duftstoffe können erfindungsgemäß einzelne Riechstoffverbindungen, z. B. die synthetischen Produkte vom Typ der Ester, Ether, Aldehyde, Ketone, Alkohole und Kohlenwasserstoffe sein. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmecyclat. Zu den Ethern zählen beispielsweise Benzylethylether und Ambroxan , zu den Aldehyden z.B. die linearen Alkanale mit 8 - 18 C-Atomen, Citral, Citronellal, Citronellyloxy-acetaldehyd, Cyclamenaldehyd, Lilial und Bourgeonal, zu den Ketonen z.B. die Jonone, alpha-Isomethylionon und Methylcedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnote erzeugen.

Solche Parfümöle können auch natürliche Riechstoffgemische enthalten, wie sie aus pflanzlichen Quellen zugänglich sind, z.B. Pine-, Citrus-, Jasmin-, Patchouly-, Rosen- oder Ylang-Ylang-Öl. Ebenfalls geeignet sind Muskateller-Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliöl, Orangenschalenöl und Sandelholzöl.

Um wahrnehmbar zu sein, muss ein Riechstoff flüchtig sein, wobei neben der Natur der funktionellen Gruppen und der Struktur der chemischen Verbindung auch die Molmasse eine wichtige Rolle spielt. So besitzen die meisten Riechstoffe Molmassen bis etwa 200 Dalton, während Molmassen von 300 Dalton und darüber eher eine Ausnahme darstellen. Aufgrund der unterschiedlichen Flüchtigkeit von Riechstoffen verändert sich der Geruch eines aus mehreren Riechstoffen zusammengesetzten Parfüms bzw. Duftstoffs während des Verdampfens, wobei man die Geruchseindrücke in "Kopfnote" (top note), "Herz- bzw. Mittelnote" (middle note bzw. body) sowie "Basisnote" (end note bzw. dry out) unterteilt. Da die Geruchswahrnehmung zu einem großen Teil auch auf der Geruchsintensität beruht, besteht die Kopfnote eines Parfüms bzw. Duftstoffs nicht allein aus leichtflüchtigen Verbindungen, während die Basisnote zum größten Teil aus weniger flüchtigen, d.h. haftfesten Riechstoffen besteht. Bei der Komposition von Parfüms können leichter flüchtige Riechstoffe beispielsweise an bestimmte Fixative gebunden werden, wodurch ihr zu schnelles Verdampfen verhindert wird. Bei der nachfolgenden Einteilung der Riechstoffe in "leichter flüchtige" bzw. "haftfeste" Riechstoffe ist also über den Geruchseindruck und darüber, ob der entsprechende Riechstoff als Kopf- oder Herznote wahrgenommen wird, nichts ausgesagt. Haftfeste Riechstoffe, die im Rahmen der vorliegenden Erfindung einsetzbar sind, sind beispielsweise die ätherischen Öle wie Angelikawurzelöl, Anisöl, Arnikablütenöl, Basilikumöl, Bayöl, Bergamottöl, Champacablütenöl, Edeltannenöl, Edeltannenzapfenöl, Elemiöl, Eukalyptusöl, Fenchelöl, Fichtennadelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaïvabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Neroliöl, Niaouliöl, Olibanumöl, Orangenöl, Origanumöl, Palmarosaöl, Patschuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-Öl, Ysop-Öl, Zimtöl, Zimtblätteröl, Zitronellöl, Zitronenöl sowie Zypressenöl. Aber auch die höhersiedenden bzw. festen Riechstoffe natürlichen oder synthetischen Ursprungs können im Rahmen der vorliegenden Erfindung als haftfeste Riechstoffe bzw. Riechstoffgemische, also Duftstoffe, eingesetzt werden. Zu diesen Verbindungen zählen die nachfolgend genannten Verbindungen sowie Mischungen aus diesen: Ambrettolid, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylakohol, Benzylacetat, Benzylbenzoat, Benzylformiat, Benzylvalerianat, Borneol, Bornylacetat, α-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p-Kresolmethylether, Cumarin, p-Methoxyacetophenon, Methyl-n-amylketon, Methylanthranilsäuremethylester, p-Methylacetophenon, Methylchavikol, p-Methylchinolin, Methyl-β-naphthylketon, Methyl-n-nonylacetaldehyd, Methyl-n-nonylketon, Muskon, β-Naphtholethylether, β-Naphtholmethylether, Nerol, Nitrobenzol, n-Nonylaldehyd, Nonylakohol, n-Octylaldehyd, p-Oxy-Acetophenon, Pentadekanolid, β-Phenylethylakohol, Phenylacetaldehyd-Dimethyacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, γ-Undelacton, Vanilin, Veratrumaldehyd, Zimtaldehyd, Zimatalkohol, Zimtsäure, Zimtsäureethylester, Zimtsäurebenzylester.

Zu den leichter flüchtigen Riechstoffen zählen insbesondere die niedriger siedenden Riechstoffe natürlichen oder synthetischen Ursprungs, die allein oder in Mischungen eingesetzt werden können. Beispiele für leichter flüchtige Riechstoffe sind Alkylisothiocyanate (Alkylsenföle), Butandion, Limonen, Linalool, Linaylacetat und -propionat, Menthol, Menthon, Methyl-n-heptenon, Phellandren, Phenylacetaldehyd, Terpinylacetat, Zitral, Zitronellal.

Besonders bevorzugte erfindungsgemäße Antitranspirant-Zusammensetzungen sind dadurch gekennzeichnet, dass mindestens eine Duftstoffkomponente in einer Gesamtmenge von 0,00001 bis 4 Gew.-%, bevorzugt 0,5 - 2 Gew.-%, jeweils bezogen auf die Gesamtzusammensetzung, enthalten ist.

Die wäßrige bzw. protische Suspension mindestens eines Antitranspirans ist zusammen mit einem Treibmittel in der erfindungsgemäßen Kombination in einem Spender enthalten, der einen Druckbehälter aus Metall umfaßt, welcher eine Pulverlack-Innenbeschichtung aufweist.

Geeignete Metalldosen sind insbesondere aus Aluminium oder Weißblech gefertigt. Eine bevorzugte Ausführungsform der vorliegenden Erfindung ist daher eine erfindungsgemäße Kombination, bei der der Spender einen Druckbehälter aus Weißblech umfaßt.

Ebenfalls bevorzugt sind erfindungsgemäße Kombinationen, bei denen der Spender einen Druckbehälter aus Aluminium umfaßt.

Der Druckbehälter weist materialunabhängig vorzugsweise bestimmte Wandstärken auf, um den Innendruck sicher tragen zu können. Hier sind erfindungsgemäße Kombinationen bevorzugt, bei denen der nicht lackierte Druckbehälter Wanddicken von 0,1 bis 1,5 mm, vorzugsweise von 0,1 bis 1 mm, weiter bevorzugt von 0,2 bis 0,75 mm und insbesondere von 0,25 bis 0,499 mm aufweist.

Unabhängig vom Material /Aluminium oder Weißblech) und von der Materialalstärke sind erfindungsgemäße Kombinationen bevorzugt, bei denen die die Pulverlack-Innenbeschichtung eine Schichtdicke von 20 bis 300 µm, vorzugsweise von 25 bis 250 µm, weiter bevorzugt von 30 bis 225 µm und insbesondere von 50 bis 200 µm aufweist.

Diese Schichtdicken haben sich in Kombination mit dem höheren Wassergehalt (bzw. Gehalt an protischen Lösungsmitteln) als besonders resistent erwiesen.

Erfindungsgemäß weisen die Druckbehälter der erfindungsgemäßen Kombination eine Pulverlack-Innenbeschichtung auf. Pulverlacke sind nach DIN EN 971-1: 1996-09 pulverförmige, lösemittelfreie Beschichtungsstoffe, die nach dem Schmelzen und gegebenenfalls Einbrennen eine Beschichtung ergeben. Sie werden in Pulverform vorwiegend auf metallische Substrate aufgetragen. Sie dienen - genau wie Naßlacke - sowohl der dekorativen Verschönerung eines Objektes als auch dem rein funktionellen Schutz einer Oberfläche. Im heutigen Sprachgebrauch versteht man unter Pulverlacken die duroplastischen Systeme. Auf thermoplastische Pulverlacke wird im Allgemeinen explizit hingewiesen. Die filmbildende Phase der Pulverlacke setzt sich wie die der Naßlacke aus Bindemitteln, Reaktionspartnern für diese Bindemittel (Härter), Füllstoffen, Pigmenten und Additiven zusammen. Die verwendeten Bindemittel und Härter bestimmen dabei im Wesentlichen die generellen Eigenschaften des Pulverlacks und damit auch sein bevorzugtes Anwendungsgebiet.

Pulverlacke basieren auf den Polymerklassen Epoxidharze, Epoxidharz/Polyester-Gemische, Polyester, Polyester/Isocyanat-Gemische und Acrylate. Die daraus gefertigten Pulverlacke heißen Epoxidharz-, Epoxidharz/Polyester-, Polyester-, Polyurethan- bzw. Acrylat-Pulverlacke.

Zur Namensgebung der Pulverlacktypen werden die zugrunde liegenden Polymerklassen ebenso herangezogen wie die Vernetzungschemie (Polyurethan-Pulverlack). Daraus resultieren manchmal irreführende Bezeichnungen. Es heißen z. B. Isocyanat-gehärtete Acrylate weiterhin Acrylate, während Isocyanat-gehärtete Polyester als Polyurethane bezeichnet werden.

Strahlenhärtende Pulverlacke runden die Palette der genannten, rein thermisch vernetzenden ab: Dabei sind die Nah-Infrarot härtenden NIR-Pulverlacke im Prinzip noch zu den thermisch härtenden zu zählen, denn sie können auch im Ofen gehärtet werden. Die UV-härtenden dagegen basieren auf einem rein strahlungshärtenden Bindemittel, benötigen also UV-Strahlung.

*Füllstoffe* beeinflussen den fertigen Lackfilm vor allem in seinen mechanischen Eigenschaften, also hinsichtlich Elastizität und Schlagtiefungsbeständigkeit sowie in der Chemikalienbeständigkeit. Aber auch Verlauf und Glanz und damit die dekorativen Aspekte lassen sich dadurch in breiten Grenzen steuern. Außerdem werden durch Füllstoffe unter anderem das Ausmaß der Kantendeckung und das Ablaufverhalten an den Kanten während des Beschichtens sowie die Dichte des Pulverlacks und damit seine Ergiebigkeit beeinflußt. Als Pulverlackfüllstoffe dienen vorwiegend natürliche Mineralien wie Schwerspate, Feldspäte und Kreiden.

Pulverlacke sind grundsätzlich in fast allen Farbtönen herstellbar. Für die Farbgebung sind dafür sowohl organische als auch anorganische Pigmente geeignet, sofern diese hinreichend temperaturstabil sind. Verwendet man als Pigmente Metalloxide, so übernehmen sie neben ihrer farbgebenden Funktion gelegentlich auch noch die Aufgabe eines Korrosionsinhibitors. Metall- und andere Effektpigmente können unter der hohen Scherbelastung bei der Herstellung von Pulverlacken leiden, so daß sie meist nach der Extrudierung in Pulverform eingearbeitet werden.

Die Pulverlackadditive umfassen Verlaufhilfsmittel zur Steuerung der Oberflächenglätte, Entgasungsmittel zur Verbesserung des Entweichens eingeschlossener Luft, Textur- und Strukturhilfsmittel zur Einstellung bestimmter Oberflächeneffekte und Mattierungsmittel. Daneben kommen Katalysatoren zur Verbesserung der Reaktivität sowie Rieselhilfen zur Beeinflussung der Rieselfähigkeit zum Einsatz.

Die Pulverlackbeschichtung in der erfindungsgemäßen Kombination kann aus unterschiedlichen Pulverlacken bestehen. Eine bevorzugte Ausführungsform der erfindungsgemäßen Kombination ist **dadurch gekennzeichnet, daß** die Pulverlack-Innenbeschichtung mindestens einen Epoxy-Pulverlack umfaßt.

Epoxidharz-Pulverlacke oder Epoxi-Pulverlacke sind Pulverlacke, in denen Epoxidharze oder epoxidierte Novolacke mit unterschiedlichen Härtern zu einer Beschichtung reagieren. Als Härter kommen je nach Anwendungsbereich Amine im weitesten Sinne oder modifiziertes Cyanoguanidin, Phenole wie Bisphenol A bzw. F oder ihre Derivate sowie Anhydrid-Härter bzw. zum Einsatz. Außer mit diesen niedermolekularen Härtern werden Epoxidharze auch mit sauren Polyesterharzen kombiniert, siehe weiter unten (Epoxidharz/Polyester-Pulverlacke).

Beschichtungen, die aus Epoxidharz-Pulverlacken hergestellt werden, zeichnen sich durch extrem guten Korrosionsschutz und gute mechanisch-technologische Werte aus.

Ein Nachteil der Epoxidharz-Pulverlacke liegt in ihrer Anfälligkeit für Vergilbung beim Überbrennen und ihrer geringen Kreidungsresistenz (siehe Kreidung). Die Einwirkung von UV-Licht führt zur Vermattung der Oberflächen, die aber die Funktionalität nicht beeinträchtigt. Für dekorative Anwendungen sind Epoxidharz-Pulverlacke aufgrund dieser Eigenschaften nur in Innenräumen geeignet.

Die Auswahl des Härters für die Vernetzung wird durch den speziellen Anwendungsfall bestimmt. Mit phenolischen Härtern werden extrem niedrige Einbrenntemperaturen erreicht. Amine, speziell die Dicyandiamid-Derivate, führen zu einer hohen Vernetzungsdichte des Pulverlackes und damit im Allgemeinen auch zu einer besonders guten Lösemittel- und Chemikalienbeständigkeit. Für Anwendungen, in denen eine hohe Glasübergangstemperatur des vernetzten Films gefordert wird, eignen sich bevorzugt Anhydride. Diese sind in den meisten Fällen stark reizend und die daraus hergestellten Pulverzubereitungen kennzeichnungspflichtig. Ihr Einsatz erfolgt daher erfindungsgemäß weniger bevorzugt.

Hervorragend geeignet sind Epoxi-Pulverlacke für den Schutz vor Korrosion mittels eines Mehrschicht-Aufbaus. Reine Epoxi-Pulverlacke zeichnen sich durch eine hervorragende Chemikalienbeständigkeit aus. Weiterhin besitzen sie eine hohe Isolationswirkung gegen elektrischen Strom.

Eine weitere bevorzugte Ausführungsform der erfindungsgemäßen Kombination ist **dadurch gekennzeichnet, daß** die Pulverlack-Innenbeschichtung mindestens einen Polyester-Pulverlack umfaßt.

Reine Polyester-Pulverlacke genügen den Anforderungen bezüglich Witterungsstabilität. Sie sind resistenter gegen UV-Strahlung und bieten deshalb langjährigen Schutz auch im Außenbereich.

Das klassische Polyester-System ist TGIC-haltig (Triglycidylisocyanurat) und wegen seines universellen Eigenschaftsbildes sehr beliebt im Markt. Polyester/TGIC-Pulverlacke werden bei Temperaturen oberhalb 150°C eingebrannt, meist bei ca. 190°C. Aufgrund ihrer geringen Vergilbungstendenz sind sogar Temperaturen bis 290°C möglich, so daß gegebenenfalls nur wenige Sekunden Objekttemperatur zur vollständigen Vernetzung des Lackfilms ausreichen.

Da Pulverlacke, welche auf diesem Härter basieren, seit 1998 als *giftig (T)* gekennzeichnet werden müssen, werden sie in Europa fast nicht mehr eingesetzt. Global erfreuen sich diese Systeme aber nach wie vor großer Beliebtheit.

Erfindungsgemäß bevorzugte Kombinationen sind **dadurch gekennzeichnet, daß** die Pulverlack-Innenbeschichtung mindestens einen Triglycidylisocyanurat-härtenden Polyester-Pulverlack umfaßt.

Bei den Ersatzsystemen dominieren zwei unterschiedliche Vernetzungsmechanismen.

Zum einen die auf der Basis einer Polykondensation mit Hydroxylalkylamid vernetzenden Systeme, die sich durch geringe Einbrenntemperaturen und einen glatten Verlauf auszeichnen. Nachteilig ist jedoch die ausgeprägte Neigung zu Nadelstichen bei höheren Schichtdicken, die aus der Vernetzungsart (Abspaltung von Wasser) resultiert. Zum anderen sind seit einiger Zeit direkte Nachfolgetypen von TGIC im Einsatz. Wie TGIC-haltige Produkte vernetzen diese mittels einer Polyaddition mit dem Polyesterharz. So werden keine Abspaltprodukte freigesetzt, welche Nadelstiche verursachen können. Nachteilig sind hier jedoch die etwas höheren Preise, ein schlechterer Verlauf (was weitere Optimierungsschritte erfordert) und eine Kennzeichnung des Pulvers als *reizend (Xi)* ab einer bestimmten Härterkonzentration im Lack.

Ganz besonders bevorzugte erfindungsgemäße Kombinationen sind **dadurch gekennzeichnet, daß** die Pulverlack-Innenbeschichtung mindestens einen Hydroxylalkylamid vernetzenden Polyester-Pulverlack umfaßt.

Eine weitere bevorzugte Ausführungsform der erfindungsgemäßen Kombination ist **dadurch gekennzeichnet, daß** die Pulverlack-Innenbeschichtung mindestens einen Polyurethan-Pulverlack umfaßt.

PU-Pulverlacke sind Pulverlacke, in denen Hydroxy-Gruppen-tragende Polyester mit Isocyanaten gehärtet werden. Prinzipiell ist jedoch auch jedes andere Hydroxy-Gruppen-tragende Polymer als Bindemittel geeignet. Die Isocyanate sind meistens verkappt (blockiert), um einerseits einen leicht handhabbaren Feststoff einsetzen zu können, andererseits, um eine Vorreaktion von Bindemittel und Härter im Pulverlack zu vermeiden. Des Weiteren gewährleisten die Verkappungsmittel einen gefahrlosen Umgang mit dem Polyurethan-Pulverlack, da sie keinerlei freie Isocyanate enthalten. Die Verkappungsmittel - meistens Caprolactam - werden beim Einbrennen erst bei Temperaturen oberhalb 175°C abgespalten, so daß für die Vernetzung von Polyurethan-Pulverlack Objekttemperaturen von mindestens 180°C notwendig sind. Da die freigesetzten Verkappungsmittel ca. 5 % der eingesetzten Pulverlackmenge ausmachen, ist beim Einsatz solcher Pulverlacke auf eine effektive Abluftreinigung zu achten.

Polyurethan-Pulverlacke ergeben witterungs- und kreidungsbeständige Lackierungen, die bevorzugt für Außenanwendungen (Fassadenelemente, Automobilanbauteile) zum Einsatz kommen. Polyurethan-Pulverlacke zeichnen sich gegenüber Polyester/TGIC-Pulverlacken durch einen deutlich besseren Verlauf und noch bessere Beständigkeiten aus. In vielen Fällen ist eine spiegelglatte Oberfläche zu erzielen. Sie sind sehr gut mattierbar. Die Mattierung wird durch Unverträglichkeit zweier unterschiedlich reaktiver Pulver hervorgerufen, indem ein langsamer Polyurethan-Pulverlack mit einem hochreaktiven Polyester/TGIC-Pulverlack gemischt wird.

Eine weitere bevorzugte Ausführungsform der erfindungsgemäßen Kombination ist **dadurch gekennzeichnet, daß** die Pulverlack-Innenbeschichtung mindestens einen Acrylat-Pulverlack umfaßt.

Acrylat-Pulverlacke sind alle Pulverlacke mit einem Acrylatharz-Anteil im Bindemittel, wobei die Vernetzungschemie nicht in die Namensgebung eingeht. Prinzipiell sind auch bei den Acrylat-Pulverlacken alle Reaktionen denkbar, die zum Aufbau eines Netzwerkes befähigt sind, nämlich Epoxid/Hydroxy-, Epoxid/Carboxy- und Hydroxy/Isocyanat-Reaktionen.

Aufgrund der chemischen Umgebung in Acrylatharzen sind für den Epoxid/Carboxy-Mechanismus, anders als bei den Epoxidharz/Polyester-Pulverlacken, Einbrenntemperaturen von 130°C ausreichend. Zwecks besserer Pulverlagerstabilität und höherer Arbeitsgeschwindigkeit wird aber meist bei 160-190°C eingebrannt. Die Isocyanat-vernetzenden Acrylat-Pulverlacke benötigen wie die Polyurethan-Pulverlacke auf Polyester-Basis 180°C Objekttemperatur.

Die Acrylat-Pulverlacke sind extrem wetter- und kratzfest und bieten auch bei niedrigen Einbrenntemperaturen speziell in transparenten Formulierungen einen sehr guten Verlauf. Die für Pulverlacke typische Orangenschalenstruktur ist auf ein Minimum reduziert. Die korrosionsschützende Wirkung ist etwas schwächer als die der Polyester/TGIC-Pulverlacke. Daher kommen die Acrylat-Pulverlacke in erster Linie nur für die Beschichtung von Aluminiumteilen oder als Deckschicht bei einer Mehrschichtlackierung in Frage.

Eine weitere bevorzugte Ausführungsform der erfindungsgemäßen Kombination ist **dadurch gekennzeichnet, daß** die Pulverlack-Innenbeschichtung mindestens einen Hybridpulverlack aus miteinander vernetzenden Epoxid- und Polyesterharzen umfaßt.

Bei Hybrid-Pulverlacken werden Epoxid- und Polyesterharze miteinander vernetzt. Diese Kombination öffnet einen breitgefächerten Anwendungsbereich. Die Witterungsbeständigkeit ist besser als bei reinen Epoxisystemen. Auch die Beständigkeiten gegen Chemikalien ist in vielen Fällen ausreichend. Weiterhin sind alle Glanz- und fast alle Textureinstellungen problemlos realisierbar. Die Farbtonvielfalt ist nahezu unbegrenzt einstellbar.

Ein weiterer Gegenstand der vorliegenden Erfindung ist ein Verfahren zur Behandlung von Körpergeruch, bei dem eine erfindungsgemäße treibmittelhaltige Antitranspirant-Zusammensetzung aus einer erfindungsgemäßen Kombination auf die Haut aufgetragen wird.

Bezüglich bevorzugter Ausführungsformen des erfindungsgemäßen Verfahrens gilt mutatis mutandis das zu den erfindungsgemäßen Zusammensetzungen Gesagte.

## Patentansprüche

1. Kombination aus einer treibmittelhaltigen Antitranspirant-Zusammensetzung und einem dafür geeigneten Spender, **dadurch gekennzeichnet, daß**
A) die treibmittelhaltige Antitranspirant-Zusammensetzung bezogen auf ihr Gewicht
a1) mindestens 1 Gew.-% mindestens eines Antitranspirant-Wirkstoffes enthält,
a2) mindestens 10 Gew.-% eines oder mehrerer protischer Lösungsmittel enthält,
a3) 5 bis 60 Gew.-% Treibmittel enthält, und
B) der Spender einen Druckbehälter aus Metall umfaßt, welcher eine Pulverlack-Innenbeschichtung aufweist.

2. Kombination nach Anspruch 1, **dadurch gekennzeichnet, daß** der Spender einen Druckbehälter aus Weißblech umfaßt.

3. Kombination nach Anspruch 1, **dadurch gekennzeichnet, daß** der Spender einen Druckbehälter aus Aluminium umfaßt.

4. Kombination nach einem der Ansprüche 1 bis 3, **dadurch gekennzeichnet, daß** der nicht lackierte Druckbehälter Wanddicken von 0,1 bis 1,5 mm, vorzugsweise von 0,1 bis 1 mm, weiter bevorzugt von 0,2 bis 0,75 mm und insbesondere von 0,25 bis 0,499 mm aufweist.

5. Kombination nach einem der Ansprüche 1 bis 4, **dadurch gekennzeichnet, daß** die Pulverlack-Innenbeschichtung eine Schichtdicke von 20 bis 300 µm, vorzugsweise von 25 bis 250 µm, weiter bevorzugt von 30 bis 225 µm und insbesondere von 50 bis 200 µm aufweist.

6. Kombination nach einem der Ansprüche 1 bis 5, **dadurch gekennzeichnet, daß** die Pulverlack-Innenbeschichtung mindestens einen Epoxy-Pulverlack umfaßt.

7. Kombination nach einem der Ansprüche 1 bis 6, **dadurch gekennzeichnet, daß** die Pulverlack-Innenbeschichtung mindestens einen Polyester-Pulverlack umfaßt.

8. Kombination nach Anspruch 7, **dadurch gekennzeichnet, daß** die Pulverlack-Innenbeschichtung mindestens einen Triglycidylisocyanurat-härtenden Polyester-Pulverlack umfaßt.

9. Kombination nach Anspruch 7, **dadurch gekennzeichnet, daß** die Pulverlack-Innenbeschichtung mindestens einen Hydroxylalkylamid vernetzenden Polyester-Pulverlack umfaßt.

10. Kombination nach einem der Ansprüche 1 bis 9, **dadurch gekennzeichnet, daß** die Pulverlack-Innenbeschichtung mindestens einen Polyurethan-Pulverlack umfaßt.

11. Kombination nach einem der Ansprüche 1 bis 10, **dadurch gekennzeichnet, daß** die Pulverlack-Innenbeschichtung mindestens einen Acrylat-Pulverlack umfaßt.

12. Kombination nach einem der Ansprüche 1 bis 11, **dadurch gekennzeichnet, daß** die Pulverlack-Innenbeschichtung mindestens einen Hybridpulverlack aus miteinander vernetzenden Epoxid- und Polyesterharzen umfaßt.

13. Kombination nach einem der Ansprüche 1 bis 12, **dadurch gekennzeichnet, daß** die treibmittelhaltige Antitranspirant-Zusammensetzung bezogen auf ihr Gewicht mindestens einen Antitranspirant-Wirkstoff in einer Gesamtmenge von 3 - 25 Gew.-%, vorzugsweise 5 - 22 Gew.-% und insbesondere 10 - 20 Gew.-%, bezogen auf das Gesamtgewicht der Aktivsubstanz in der gesamten Zusammensetzung, enthält.

14. Kombination nach einem der Ansprüche 1 bis 13, **dadurch gekennzeichnet, daß** die treibmittelhaltige Antitranspirant-Zusammensetzung bezogen auf ihr Gewicht 10 bis 80 Gew.-%, vorzugsweise 12,5 bis 75 Gew.-%, weiter bevorzugt 15 bis 70 Gew.-%, noch weiter bevorzugt 20 bis 65 Gew.-%, besonders bevorzugt 22,5 bis 62,5 Gew.-% und insbesondere 25 bis 60 Gew.-% mindestens eines protischen Lösungsmittels, ausgewählt aus Ethanol, Wasser und deren Mischungen, enthält.

15. Kombination nach einem der Ansprüche 1 bis 14, **dadurch gekennzeichnet, daß** die treibmittelhaltige Antitranspirant-Zusammensetzung bezogen auf ihr Gewicht 7,5 bis 57,5 Gew.-%, vorzugsweise 10 bis 55 Gew.-%, weiter bevorzugt 15 bis 52,5 Gew.-%, noch weiter bevorzugt 17,5 bis 55 Gew.-%, besonders bevorzugt 20 bis 50 Gew.-% und insbesondere 25 bis 45 Gew.-% mindestens eines Treibmittels, ausgewählt aus n-Propan, n-Butan, iso-Butan, n-Pentan, Dimethylether und deren Mischungen, enthält.
